# EUROPEAN PATENT APPLICATION

(11) **EP 1 939 200 A1**
(43) Date of publication of application: **02.07.2008**
(21) Application number: 06798242.1
(22) Date of filing: 22.09.2006
(51) Int. Cl.: C07D 473/18, A61K 31/522, A61P 11/02, A61P 11/06, A61P 17/00, A61P 27/02, A61P 31/12, A61P 31/18, A61P 35/00, A61P 37/02, A61P 37/08, A61P 43/00, C07D 473/16, C07D 473/24, C07D 473/34

(54) **NOVEL ADENINE COMPOUND**

(30) Priority: 22.09.2005 JP 2005275092
(71) Applicant: Dainippon Sumitomo Pharma Co., Ltd., Osaka 541-8524 (JP); AstraZeneca AB, 151 85 Södertälje (SE)
(72) Inventor: MILLICHIP, Ian, Leicestershire LE115RH (GB); MCINALLY, Thomas, Leicestershire LE115RH (GB); BONNERT, Roger, Leicestershire LE115RH (GB)
(74) Representative: Vossius & Partner
(86) International application number: PCT/JP2006/318854
(87) International publication number: WO 2007/034916

(57) **Abstract**

A novel adenine compound represented by the formula (1): 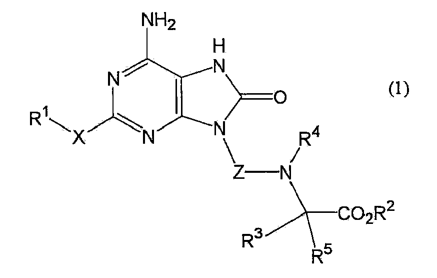 wherein Z represents (un)substituted alkylene, a single bond, etc.; R¹ represents an (un)substituted alkyl, alkenyl, alkynyl, cycloalkyl, aryl, or heteroaryl group; R² represents hydrogen or (un)substituted alkyl; R³, R⁴ and R⁵ each independently represents an (un)substituted alkyl, alkenyl, alkynyl, aryl, or heteroaryl group, provided that R³ and R⁵ may be bonded to each other to form a 3- to 7-membered, saturated carbocycle or heterocycle in cooperation with the adjacent carbon atom; and X represents oxygen, sulfur, SO₂, NR⁶ (R⁶ represents hydrogen or alkyl), or a single bond, or a pharmaceutically acceptable salt of the compound. The compound and salt are useful as a medicine.

## Description

### TECHNICAL FIELD

The present invention relates to a novel adenine compound useful as a therapeutic and/or preventive agent for allergic disease, viral disease or cancer, etc.

### BACKGROUND ART

In case that foreign substances including bacteria, virus or parasite invade living organisms, immune systems exist in order to exclude said substances. In acquired immune systems, antigen processing by antigen presenting cells such as dendritic cells (DCs) is carried out when the foreign substances invade, and naive Th cells functionally differentiate via interactions of DCs/Th cells into Th1 cells or Th2 cells which play a central role of immune response in vivo. It is believed that immune diseases are developed by one-way deflection of immuno-balance of Th 1 cells or Th2 cells in this process.

Specifically, cytokine such as interleukin-4 (IL-4) and interleukin-5 (IL-5) secreted by Th2 cells is secreted in an excess amount within the body of patients with allergic diseases, and the compound inhibiting immune response of Th2 cells may be expected to be a therapeutic agent for allergic disease. Also, the compound enhancing immune response of Th 1 cells may be expected to be a therapeutic or preventive agent for viral disease or cancer.

In the meantime, it was believed until recently that natural immune system was caused by nonspecific phagocytosis, but it was proved that Toll-like receptor (TLR) exists and principal parts of natural immunity activation are carried out via TLR. Moreover, a ligand of TLR may be expected to have a function as a Th1/Th2 differentiation controlling agent and to be useful for treatment or prevention of immune diseases in that TLR recognizes a ligand to induce inflammatory cytokine such as IL-12, TNF, and IL-12 differentiates and induces naive T cell to Th1 cell. Actually, it is known that Th2 cell predominates in patients with asthma, atopic dermatitis, etc., and asthma-targeted clinical trials are carried out for DNA (CpGDNA) derived from microorganism, TLR9 agonist. Additionally, it is known that TLR7/8 agonist imidazoquinoline derivative (see Patent Document 1) also shows producing inhibitory activity of Th2 cytokine interleukin-4 (IL-4) and interleukin-5 (IL-5), and is actually useful for allergic diseases in animal models.

Meanwhile, compounds described in, for example, Patent Documents 2 to 4 are known as compounds with adenine skeletons which are effective for immune diseases such as viral diseases and allergic diseases.
Patent Document 1: US Patent No. 4689338
Patent Document 2: WO98/01448
Patent Document 3: WO99/28321
Patent Document 4: WO04/029054

### DISCLOSURE OF INVENTION

### Problems to be Resolved by the Invention

Problems to be resolved by the invention are directed to provide a TLR activator, more particularly a novel adenine compound which activates as a TLR7 activator, and an immune-regulating agent comprising the same as an active ingredient, for example, a therapeutic or preventive agent for allergic disease such as asthma, COPD, allergic rhinitis, allergic conjunctivitis or atopic dermatitis, viral disease such as hepatitis B, hepatitis C, HIV or HPV, bacterial infectious disease, cancer or dermatitis, etc.

### Means of Solving the Problems

The present inventors found the novel adenine compounds of the present invention according to their intensive study in order to obtain a therapeutic or preventive agent for immune diseases such as allergic disease, viral disease or cancer with excellent TLR activating effect. In other words, the compounds of the present invention are effective as a therapeutic or preventive agent for allergic disease, viral disease, or cancer, etc.

The present invention has been achieved on the basis of the above knowledge. Specifically, the present invention relates to the following inventions.
[1] An adenine compound of the formula (1): 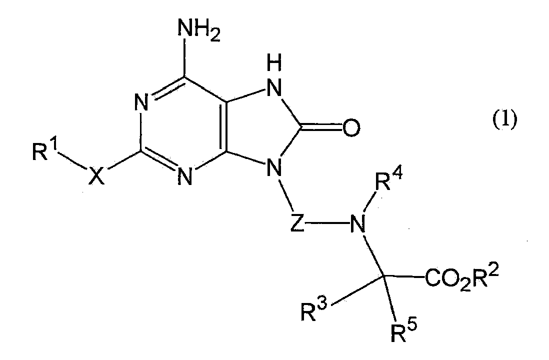
   wherein Z is substituted or unsubstituted alkylene, or a single bond, in which any 1 to 3 of methylene group(s) in the alkylene may be replaced by oxygen, sulfur, SO, SO₂ or carbonyl groups;
   R¹ is substituted or unsubstituted alkyl, substituted or unsubstituted alkenyl, substituted or unsubstituted alkynyl, substituted or unsubstituted cycloalkyl, substituted or unsubstituted aryl, or substituted or unsubstituted heteroaryl group;
   R² is hydrogen, or substituted or unsubstituted alkyl group;
   R³, R⁴ and R⁵ are independently hydrogen, substituted or unsubstituted alkyl, substituted or unsubstituted alkenyl, substituted or unsubstituted alkynyl, substituted or unsubstituted aryl, or substituted or unsubstituted heteroaryl, or R³ and R⁵ may be combined together with the adjacent carbon atom to form substituted or unsubstituted 3- to 7-membered saturated carbocycle or heterocycle;
   X is oxygen, sulfur, SO₂, NR⁶, wherein R⁶ is hydrogen or alkyl group, or a single bond; or a pharmaceutically acceptable salt thereof.

[2] The adenine compound of [1], wherein X is oxygen, sulfur, SO₂, NR⁶, wherein R⁶ is hydrogen or alkyl group with 1 to 6 carbon atom(s), or a single bond;
Z is alkylene with 2 to 6 carbon atoms wherein the alkylene may be substituted with halogen, hydroxyl, alkoxy with 1 to 6 carbon atom(s), 6- to 10-membered aryl or 5- to 10-membered heteroaryl, wherein the aryl group and the heteroaryl group may be substituted with 1 or more substituent(s) independently selected from halogen, hydroxyl, alkyl with 1 to 6 carbon atom(s), alkoxy with 1 to 6 carbon atom(s), haloalkyl with 1 to 6 carbon atom(s), haloalkoxy with 1 to 6 carbon atom(s), and amino optionally substituted with the same or different 1 or 2 alkyl(s) with 1 to 6 carbon atom(s);
R¹ is substituted or unsubstituted alkyl with 1 to 6 carbon atom(s), substituted or unsubstituted alkenyl with 2 to 6 carbon atoms, substituted or unsubstituted alkynyl with 2 to 6 carbon atoms, substituted or unsubstituted 6- to 10-membered aryl, substituted or unsubstituted 5- to 10-membered heteroaryl, or substituted or unsubstituted 3- to 8-membered cycloalkyl;
R² is hydrogen, or substituted or unsubstituted alkyl with 1 to 6 carbon atom(s);
R³, R⁴ and R⁵ are independently hydrogen, substituted or unsubstituted alkyl with 1 to 6 carbon atom(s), substituted or unsubstituted alkenyl with 2 to 6 carbon atoms, substituted or unsubstituted alkynyl with 2 to 6 carbon atoms, substituted or unsubstituted 6- to 10-membered aryl, or substituted or unsubstituted 5- to 10-membered heteroaryl, or R³ and R⁵ may be combined together with the adjacent carbon atom to form substituted or unsubstituted 3-to 7-membered saturated carbocycle, or substituted or unsubstituted 4-to 7-membered saturated nitrogen-containing heterocycle;
the substituted alkyl, substituted alkenyl and substituted alkynyl are substituted with 1 or more substituent(s) independently selected from the following (a) to (c):
(a) halogen, hydroxyl, carboxy, haloalkyl with 1 to 6 carbon atom(s), haloalkoxy with 1 to 6 carbon atom(s), mercapto;
(b) alkoxy with 1 to 6 carbon atom(s), alkylthio with 1 to 6 carbon atom(s), alkylcarbonyl with 2 to 6 carbon atoms, alkylcarbonyloxy with 2 to 6 carbon atoms, alkoxycarbonyl with 2 to 6 carbon atoms, alkylsulfonyl with 1 to 6 carbon atom(s), alkylsulfinyl with 1 to 6 carbon atom(s), alkenyloxy with 2 to 6 carbon atoms, alkenylthio with 2 to 6 carbon atoms, alkenylcarbonyl with 3 to 6 carbon atoms, alkenylcarbonyloxy with 3 to 6 carbon atoms, alkenyloxycarbonyl with 3 to 6 carbon atoms, alkenylsulfonyl with 2 to 6 carbon atoms, alkenylsulfinyl with 2 to 6 carbon atoms, alkynyloxy with 2 to 6 carbon atoms, alkynylthio with 2 to 6 carbon atoms, alkynylcarbonyl with 3 to 6 carbon atoms, alkynylcarbonyloxy with 3 to 6 carbon atoms, alkynyloxycarbonyl with 3 to 6 carbon atoms, alkynylsulfonyl with 2 to 6 carbon atoms, alkynylsulfinyl with 2 to 6 carbon atoms,
   wherein each group may further be substituted by 1 or more substituent(s) independently selected from halogen, hydroxyl, alkoxy with 1 to 6 carbon atom(s), carboxy, alkoxycarbonyl with 2 to 6 carbon atoms, alkylsulfonyl with 1 to 6 carbon atom(s), amino optionally substituted by the same or different 1 or 2 alkyl(s) with 1 to 6 carbon atom(s), carbamoyl optionally substituted by the same or different 1 or 2 alkyl(s) with 1 to 6 carbon atom(s), and sulfamoyl optionally substituted by the same or different 1 or 2 alkyl(s) with 1 to 6 carbon atom(s);
(c) amino, carbamoyl or sulfamoyl which may be independently substituted by 1 or 2 substituent(s) described in the following (j) to (l), 6- to 10-membered aryl, 6- to 10-membered aryloxy, 6- to 10-membered arylcarbonyl, 6- to 10-membered arylcarbonyloxy, 6- to 10-membered aryloxycarbonyl, 6- to 10-membered arylsulfonyl, 6- to 10-membered arylsulfinyl, 5- to 10-membered heteroaryl, 5- to 10-membered heteroaryloxy, 5- to 10-membered heteroarylcarbonyl, 5- to 10-membered heteroarylcarbonyloxy, 5- to 10-membered heteroaryloxycarbonyl, 5- to 10-membered heteroarylsulfonyl or 5- to 10-membered heteroarylsulfinyl which may be independently substituted by 1 or more substituent(s) described in the following (g) to (i), or 3- to 8-membered cycloalkyl, 3- to 8-membered cycloalkoxy, 3- to 8-membered cycloalkylcarbonyl, 3- to 8-membered cycloalkylcarbonyloxy, 3- to 8-membered cycloalkoxycarbonyl, 3- to 8-membered cycloalkylsulfonyl, 3- to 8-membered cycloalkylsulfinyl or 4-to 8-membered saturated heterocycle which may be independently substituted by 1 or more substituent(s) described in the following (d) to (f);
the substituted cycloalkyl and the substituted 3- to 8-membered saturated carbocycle and substituted 4- to 8-membered saturated nitrogen-containing heterocycle which both of R³ and R⁵ are combined to form are substituted by 1 or more substituent(s) independently selected from the following (d) to (f):
(d) halogen, hydroxyl, carboxy, mercapto, haloalkyl with 1 to 6 carbon atom(s), haloalkoxy with 1 to 6 carbon atom(s);
(e) alkyl with 1 to 6 carbon atom(s), alkenyl with 2 to 6 carbon atoms, alkynyl with 2 to 6 carbon atoms, alkoxy with 1 to 6 carbon atom(s), alkylthio with 1 to 6 carbon atom(s), alkylcarbonyl with 2 to 6 carbon atoms, alkylcarbonyloxy with 2 to 6 carbon atoms, alkoxycarbonyl with 2 to 6 carbon atoms, alkylsulfonyl with 1 to 6 carbon atom(s), alkylsulfinyl with 1 to 6 carbon atom(s),
   wherein each group may further be substituted by 1 or more substituent(s) independently selected from halogen, hydroxyl, alkoxy with 1 to 6 carbon atom(s), carboxy, alkoxycarbonyl with 2 to 6 carbon atoms, alkylsulfonyl with 1 to 6 carbon atom(s), amino optionally substituted by the same or different 1 or 2 alkyl(s) with 1 to 6 carbon atom(s), carbamoyl optionally substituted by the same or different 1 or 2 alkyl(s) with 1 to 6 carbon atom(s), and sulfamoyl optionally substituted by the same or different 1 or 2 alkyl(s) with 1 to 6 carbon atom(s);
(f) 6- to 10-membered aryl or 5- to 10-membered heteroaryl which may be independently substituted by 1 or more substituent(s) described in the following (g) to (i), or amino, carbamoyl or sulfamoyl which may be independently substituted by 1 or 2 substituent(s) described in the following (j) to (l);
the substituted aryl and the substituted heteroaryl are substituted by 1 or more substituent(s) independently selected from the following (g) to (i):
(g) halogen, hydroxyl, mercapto, cyano, nitro, haloalkyl with 1 to 6 carbon atom(s), haloalkoxy with 1 to 6 carbon atom(s);
(h) alkyl with 1 to 6 carbon atom(s), alkoxy with 1 to 6 carbon atom(s), alkylthio with 1 to 6 carbon atom(s), alkylcarbonyl with 2 to 6 carbon atoms, alkylcarbonyloxy with 2 to 6 carbon atoms, alkylsulfonyl with 1 to 6 carbon atom(s), alkylsulfinyl with 1 to 6 carbon atom(s), alkenyl with 2 to 6 carbon atoms, alkynyl with 2 to 6 carbon atoms, 3-to 8-membered cycloalkyl, 4- to 8-membered saturated heterocycle,
   wherein each group may further be substituted by 1 or more substituent(s) independently selected from halogen, hydroxyl, alkyl with 1 to 6 carbon atom(s), alkoxy with 1 to 6 carbon atom(s), alkylsulfonyl with 1 to 6 carbon atom(s), amino optionally substituted by the same or different 1 or 2 alkyl(s) with 1 to 6 carbon atom(s), carbamoyl optionally substituted by the same or different 1 or 2 alkyl(s) with 1 to 6 carbon atom(s), and sulfamoyl optionally substituted by the same or different 1 or 2 alkyl(s) with 1 to 6 carbon atom(s);
(i) amino, carbamoyl or sulfamoyl which may be independently substituted by 1 or 2 substituent(s) described in the following (j) to (l);
the amino, carbamoyl or sulfamoyl may be substituted by 1 or 2 substituent(s) independently selected from the following (j) to (l):
(j) alkyl with 1 to 6 carbon atom(s), alkenyl with 2 to 6 carbon atoms, alkynyl with 2 to 6 carbon atoms, alkylcarbonyl with 2 to 6 carbon atoms, alkoxycarbonyl with 2 to 6 carbon atoms, alkylsulfonyl with 1 to 6 carbon atom(s), alkylsulfinyl with 1 to 6 carbon atom(s), alkenylcarbonyl with 3 to 6 carbon atoms, alkenyloxycarbonyl with 3 to 6 carbon atoms, alkenylsulfonyl with 2 to 6 carbon atoms, alkenylsulfinyl with 2 to 6 carbon atoms, alkynylcarbonyl with 2 to 6 carbon atoms, alkynyloxycarbonyl with 2 to 6 carbon atoms, alkynylsulfonyl with 2 to 6 carbon atoms, alkynylsulfinyl with 2 to 6 carbon atoms, 3- to 8-membered cycloalkyl, 3- to 8-membered cycloalkylcarbonyl, 3- to 8-membered cycloalkoxycarbonyl, 3- to 8-membered cycloalkylsulfonyl, 3- to 8-membered cycloalkylsulfinyl, 4- to 8-membered saturated heterocycle,
   wherein each group may further be substituted by 1 or more substituent(s) independently selected from halogen, hydroxyl, alkoxy with 1 to 6 carbon atom(s), carboxy, alkoxycarbonyl with 2 to 6 carbon atoms, alkylsulfonyl with 1 to 6 carbon atom(s), amino optionally substituted by the same or different 1 or 2 alkyl(s) with 1 to 6 carbon atom(s), carbamoyl optionally substituted by the same or different 1 or 2 alkyl(s) with 1 to 6 carbon atom(s), and sulfamoyl with 1 to 6 carbon atom(s) optionally substituted by the same or different 1 or 2 alkyl(s);
(k) 6- to 10-membered aryl, 6- to 10-membered arylcarbonyl, 6- to 10-membered aryloxycarbonyl, 6- to 10-membered arylsulfonyl, 6- to 10-membered arylsulfinyl, 5- to 10-membered heteroaryl, 5- to 10-membered heteroarylcarbonyl, 5- to 10-membered heteroaryloxycarbonyl, 5- to 10-membered heteroarylsulfonyl, 5- to 10-membered heteroarylsulfinyl,
   wherein each group may further be substituted by 1 or more substituent(s) independently selected from halogen, hydroxyl, alkyl with 1 to 6 carbon atom(s), alkoxy with 1 to 6 carbon atom(s), alkylsulfonyl with 1 to 6 carbon atom(s), amino optionally substituted by the same or different 1 or 2 alkyl(s) with 1 to 6 carbon atom(s), carbamoyl optionally substituted by the same or different 1 or 2 alkyl(s) with 1 to 6 carbon atom(s), and sulfamoyl optionally substituted by the same or different 1 or 2 alkyl(s) with 1 to 6 carbon atom(s);
(l) when 2 substituents of amino, carbamoyl and sulfamoyl are combined together with nitrogen atom to form 4- to 7-membered saturated nitrogen-containing heterocycle with 1 to 4 heteroatom(s) selected from 1 to 2 nitrogen(s), 0 to 1 oxygen and 0 to 1 sulfur,
   wherein the saturated nitrogen-containing heterocycle may be substituted on any carbon atoms or nitrogen atoms by halogen, hydroxyl, alkyl with 1 to 6 carbon atom(s), alkoxy with 1 to 6 carbon atom(s), carboxyl, alkoxycarbonyl with 2 to 6 carbon atoms, alkylsulfonyl with 1 to 6 carbon atom(s), amino optionally substituted by the same or different 1 or 2 alkyl(s) with 1 to 6 carbon atom(s), carbamoyl optionally substituted by the same or different 1 or 2 alkyl(s) with 1 to 6 carbon atom(s), or sulfamoyl optionally substituted by the same or different 1 or 2 alkyl(s) with 1 to 6 carbon atom(s), where the substituent may be kept in chemically stable state; or a pharmaceutically acceptable salt thereof.

[3] The adenine compound of either of [1] or [2], wherein R² is alkyl(s) with 1 to 4 carbon atom(s) in the formula (1), or a pharmaceutically acceptable salt thereof.

[4] The adenine compound of [3], wherein R² is methyl, or a pharmaceutically acceptable salt thereof.

[5] The adenine compound of [1], wherein R² is substituted alkyl with 2 to 6 carbon atoms in the formula (1), or a pharmaceutically acceptable salt thereof.

[6] The adenine compound of [5], wherein R² is alkyl with 2 to 6 carbon atoms substituted by substituted or unsubstituted amino in the formula (1), or a pharmaceutically acceptable salt thereof.

[7] The adenine compound of any one of [1] to [6], wherein R³ and R⁴ are independently hydrogen or alkyl with 1 to 3 carbon atom(s) in the formula (1), or a pharmaceutically acceptable salt thereof.

[8] The adenine compound of any one of [1] to [7], wherein R⁵ is hydrogen or substituted or unsubstituted alkyl in the formula (1), or a pharmaceutically acceptable salt thereof.

[9] The adenine compound of [8], wherein the substituent on the substituted alkyl in R⁵ is selected from alkoxycarbonyl with 2 to 5 carbon atoms, carboxy, hydroxyl, amino optionally substituted by the same or different 1 or 2 alkyl(s) with 1 to 6 carbon atom(s), carbamoyl and 6- to 10-membered aryl wherein the aryl may be substituted by 1 or more of the same or different substituent(s) independently selected from halogen; hydroxyl; alkyl with 1 to 6 carbon atom(s) or alkoxy with 1 to 6 carbon atom(s) each of which may be substituted by hydroxyl, alkoxy with 1 to 6 carbon atom(s) or amino optionally substituted by the same or different 1 or 2 alkyl(s) with 1 to 6 carbon atom(s); haloalkyl with 1 to 6 carbon atom(s); haloalkoxy with 1 to 6 carbon atom(s); and amino optionally substituted by the same or different 1 or 2 alkyl(s) with 1 to 6 carbon atom(s), or a pharmaceutically acceptable salt thereof.

[10] The adenine compound of [1] selected from the following compounds:
methyl N-[2-(6-amino-2-butoxy-7,8-dihydro-8-oxo-9H-purin-9-yl)-ethyl]-glycine;
methyl (2S)-2-{[2-(6-amino-2-butoxy-8-oxo-7,8-dihydro-9H-purin-9-yl)-ethyl] -amino}-butanone;
methyl N-[2-(6-amino-2-butoxy-8-oxo-7,8-dihydro-9H-purin-9-yl)-ethyl]-L-alaninate;
methyl N-[2-(6-amino-2-butoxy-8-oxo-7,8-dihydro-9H-purin-9-yl)-ethyl]-2-methylalaninate;
methyl N-[2-(b-amino-2-butoxy-8-oxo-7,8-dihydro-9H-purin-9-yl)-ethyl]-D-valinate;
dimethyl N-[2-(6-amino-2-butoxy-8-oxo-7,8-dihydro-9H-purin-9-yl)-ethyl]-L-aspartate;
N²-[2-(6-amino-2-butoxy-8-oxo-7,8-dihydro-9H-purin-9-yl)-ethyl]-L-lysine methyl ester;
methyl (2S)-{[2-(6-amino-2-butoxy-8-oxo-7,8-dihydro-9H-purin-9-yl)-ethyl]-amino}(4-hydroxyphenyl)-acetate;
methyl N-[2-(6-amino-2-butoxy-8-oxo-7,8-dihydro-9H-purin-9-yl)-ethyl]-N-methyl glycinate;
methyl N-[3-(6-amino-2-butoxy-8-oxo-7,8-dihydro-9H-purin-9-yl)-propyl]-O-[3-(dimethylamino)propyl]-L-tyrosinate;
methyl N-[4-(6-amino-2-butoxy-8-oxo-7,8-dihydro-9H-purin-9-yl)butyl]-L-alaninate;
methyl N-[4-(6-amino-2-butoxy-8-oxo-7,8-dihydro-9H-purin-9-yl)butyl]-N-methyl glycinate;
N-[2-(6-amino-2-butoxy-8-oxo-7,8-dihydro-9H-purin-9-yl)-ethyl]-L-alanine;
N-[2-(6-amino-2-butoxy-8-oxo-7,8-dihydro-9H-purin-9-yl) -ethyl] -D-valine;
N-[2-(6-amino-2-butoxy-8-oxo-7,8-dihydro-9H-purin-9-yl)-ethyl]-glycine;
(2S)-2-{[2-(6-amino-2-butoxy-8-oxo-7,8-dihydro-9H-purin-9-yl)-ethyl]-amino}butane acid;
N-[2-(6-amino-2-butoxy-8-oxo-7,8-dihydro-9H-purin-9-yl]-ethyl]-2-methylalanine;
N-[2-(6-amino-2-butoxy-8-oxo-7,8-dihydro-9H-purin-9-yl)-ethyl]-N-methylglycine; or a pharmaceutically acceptable salt thereof.

[11] A pharmaceutical composition comprising as an active ingredient the adenine compound of any one of [1] to [10], or a pharmaceutically acceptable salt thereof.

[12] An agent for increasing TLR7 activity comprising as an active ingredient the adenine compound of any one of [1] to [10], or a pharmaceutically acceptable salt thereof.

[13] An immune-regulating agent comprising as an active ingredient the adenine compound of any one of [1] to [10], or a pharmaceutically acceptable salt thereof.

[14] A therapeutic or preventive agent for allergic disease, viral disease or cancer comprising as an active ingredient the adenine compound of any one of [1] to [10], or a pharmaceutically acceptable salt thereof.

[15] A therapeutic or preventive agent for asthma, COPD, allergic rhinitis, allergic conjunctivitis, atopic dermatitis, cancer, hepatitis B, hepatitis C, HIV, HPV, bacterial infectious disease or dermatitis comprising as an active ingredient the adenine compound of any one of [1] to [10], or a pharmaceutically acceptable salt thereof.

[16] A pharmaceutical composition for local administration comprising as an active ingredient the adenine compound of any one of [1] to [10], or a pharmaceutically acceptable salt thereof.

### Effect of the Invention

The present invention enables to provide useful and novel adenine compounds as a therapeutic or preventive agent for allergic disease, viral disease or cancer, etc.

### BEST MODE FOR CARRYING OUT THE INVENTION

The embodiments of the present invention are explained in detail below.
The term "halogen" as used herein includes fluorine, chlorine, bromine or iodine, preferably fluorine or chlorine.

The term "alkyl" includes straight chain or branched chain alkyl with 1 to 12 carbon atom(s), particularly, methyl, ethyl, propyl, 1-methylethyl, butyl, 2-methylpropyl, 1-methylpropyl, 1,1-dimethylethyl, pentyl, 3-methylbutyl, 2-methylbutyl, 2,2-dimethylpropyl, 1-ethylpropyl, 1,1-dimethylpropyl, hexyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, heptyl, 1-methylhexyl, 1-ethylpentyl, octyl, 1-methylheptyl, 2-ethylhexyl, nonyl, decyl, etc. Preferable one is alkyl with 1 to 6 carbon atom(s), more preferably, alkyl with 1 to 4 carbon atom(s).

The term "alkenyl" includes straight chain or branched chain alkenyl with 2 to 10 carbon atoms, particularly, ethenyl, propenyl, 1-methylethenyl, butenyl, 2-methylpropenyl, 1-methylpropenyl, pentenyl, 3-methylbutenyl, 2-methylbutenyl, 1-ethylpropenyl, hexenyl, 4-methylpentenyl, 3-methylpentenyl, 2-methylpentenyl, 1-methylpentenyl, 3,3-dimethylbutenyl, 1,2-dimethylbutenyl, heptenyl, 1-methylhexenyl, 1-ethylpentenyl, octenyl, 1-methylheptenyl, 2-ethylhexenyl, nonenyl, decenyl, etc. Preferable one is alkenyl with 2 to 6 carbon atoms, more preferably, alkenyl with 2 to 4 carbon atoms.

The term "alkynyl" includes straight chain or branched chain alkynyl with 2 to 10 carbon atoms, particularly, ethynyl, propynyl, butynyl, pentynyl, 3-methylbutynyl, hexynyl, 4-methylpentynyl, 3-methylpentynyl, 3,3-dimethylbutynyl, heptynyl, octynyl, 3-methylheptynyl, 3-ethylhexynyl, nonynyl, decynyl, etc. Preferable one is alkynyl with 2 to 6 carbon atoms, more preferably, alkynyl with 2 to 4 carbon atoms.

The term "cycloalkyl" includes 3- to 8-membered monocyclic cycloalkyl, particularly, cyclopropyl, cyclobutyl, cyclopentyl, cyclohexyl, cycloheptyl or cyclooctyl.

The term "cycloalkoxy" includes 3- to 8-membered monocyclic cycloalkoxy, particularly, cyclopropyloxy, cyclobutyloxy, cyclopentyloxy, cyclohexyloxy, cycloheptyloxy or cyclooctyloxy.

The term "aryl" includes 6- to 10-membered aryl, particularly, phenyl, 1-naphthyl or 2-naphthyl.

The term "heteroaryl" includes 5- to 10-membered mono- or bi-cyclic heteroaryl containing 1 to 4 heteroatom(s) selected from 0 to 2 nitrogen(s), 0 to 1 oxygen and 0 to 1 sulfur, particularly, furyl, thienyl, pyrrolyl, pyridyl, indolyl, isoindolyl, quinolyl, isoquinolyl, pyrazolyl, imidazolyl, pyrimidinyl, pyrazinyl, pyridazinyl, thiazolyl, oxazolyl, etc.

The term "saturated heterocycle" includes 4- to 10-membered mono- or bi-cyclic saturated heterocycle containing 1 to 3 heteroatom(s) selected from 0 to 3 nitrogen(s), 0 to 1 oxygen and 0 to 1 sulfur, wherein sulfur may be substituted by 1 or 2 oxygen(s), particularly, pyrrolidinyl, piperidinyl, piperazinyl, morpholinyl, thiomorpholinyl, 1-oxothiomorpholinyl, 1,1-dioxothiomorpholinyl, tetrahydrofuranyl, oxazolidinyl, etc. Substituents may bind on any carbon atoms or nitrogen atoms where it may be kept in chemically stable state without any limitation for binding positions. Preferable one is 4- to 8-membered monocyclic saturated heterocycle.

The term "alkylene" includes straight chain or branched chain alkylene with 1 to 12 carbon atom(s), particularly, methylene, ethylene, trimethylene, tetramethylene, pentamethylene, hexamethylene, heptamethylene, octamethylene, nonamethylene, decamethylene, 1-methylmethylene, 1-ethylmethylene, 1-propylmethylene, 1-methylethylene, 2-methylethylene, 1-methyltrimethylene, 2-methyltrimethylene, 2-methyltetramethylene, 3-methylpentamethylene, etc. The alkylene preferably includes straight chain or branched chain alkylene with 1 to 6 carbon atom(s).

The term "haloalkyl" includes alkyl substituted by 1 to 5 of the same or different halogen(s), particularly, trifluoromethyl, 2,2,2-trifluoroethyl, 2,2-difluoroethyl, pentafluoroethyl, etc.

The term "alkoxy" includes straight chain or branched chain alkoxy with 1 to 10 carbon atom(s), particularly, methoxy, ethoxy, propoxy, 1-methylethoxy, butoxy, 2-methylpropoxy, 1-methylpropoxy, 1,1-dimethylethoxy, pentoxy, 3-methylbutoxy, 2-methylbutoxy, 2,2-dimethylpropoxy, 1-ethylpropoxy, 1,1-dimethylpropoxy, hexyloxy, 4-methylpentyloxy, 3-methylpentyloxy, 2-methylpentyloxy, 1-methylpentyloxy, 3,3-dimethylbutoxy, 2,2-dimethylbutoxy, 1,1-dimethylbutoxy, 1,2-dimethylbutoxy, heptyloxy, 1-methylhexyloxy, 1-ethylpentyloxy, octyloxy, 1-methylheptyloxy, 2-ethylhexyloxy, nonyloxy, decyloxy, etc. Preferable one is alkoxy with 1 to 6 carbon atom(s), more preferably, alkoxy with 1 to 4 carbon atom(s).

The term "haloalkoxy" includes alkoxy substituted by 1 to 5 of the same or different halogen(s), particularly, trifluoromethoxy, 2,2,2-trifluoroethoxy, 2,2-difluoroethoxy, 2-fluoroethoxy, pentafluoroethoxy, etc.

The term "alkylthio" includes straight chain or branched chain alkylthio with 1 to 10 carbon atom(s), particularly, methylthio, ethylthio, propylthio, 1-methylethylthio, butylthio, 2-methylpropylthio, 1-methylpropylthio, 1,1-dimethylethylthio, pentylthio, 3-methylbutylthio, 2-methylbutylthio, 2,2-dimethylpropylthio, 1-ethylpropylthio, 1,1-dimethylpropylthio, hexylthio, 4-methylpentylthio, 3-methylpentylthio, 2-methylpentylthio, 1-methylpentylthio, 3,3-dimethylbutylthio, 2,2-dimethylbutylthio, 1,1-dimethylbutylthlo, 1,2-dimethylbutylthio, heptylthio, 1-methylhexylthio, 1-ethylpentylthio, octylthio, 1-methylheptylthio, 2-ethylhexylthio, nonylthio, decylthio, etc. Preferable one is alkylthio with 1 to 6 carbon atom(s), more preferably, alkylthio with 1 to 4 carbon atom(s).

The term "alkyl" in "alkylcarbonyl", "alkylcarbonyloxy", "alkylsulfonyl" or "alkylsulfinyl" includes the same as the alkyl group as defined hereinbefore.

The term "alkylcarbonyl" particularly includes acetyl, propanoyl, butanoyl, 2-methylpropanoyl, pentanoyl, 3-methylbutanoyl, 2-methylbutanoyl, 2,2-dimethylpropanoyl (pivaloyl), hexanoyl, 4-methylpentanoyl, 3-methylpentanoyl, 2-methylpentanoyl, 3,3-dimethylbutanoyl, 2,2-dimethylbutanoyl, heptanoyl, octanoyl, 2-ethylhexanoyl, nonanoyl, decanoyl, etc. Preferable one is alkylcarbonyl with 2 to 6 carbon atoms, more preferably, straight chain or branched chain alkylcarbonyl with 2 to 5 carbon atoms.

The term "alkylcarbonyloxy" particularly includes acetoxy, propanoyloxy, butanoyloxy, 2-methylpropanoyloxy, pentanoyloxy, 3-methylbutanoyloxy, 2-methylbutanoyloxy, 2,2-dimethylpropanoyloxy (pivaloyloxy), hexanoyloxy, 4-methylpentanoyloxy, 3-methylpentanoyloxy, 2-methylpentanoyloxy, 3,3-dimethylbutanoyloxy, 2,2-dimethylbutanoyloxy, heptanoyloxy, octanoyloxy, 2-ethylhexanoyloxy, nonanoyloxy, decanoyloxy, etc. Preferable one is alkylcarbonyloxy with 2 to 6 carbon atoms, more preferably, straight chain or branched chain alkylcarbonyloxy with 2 to 5 carbon atoms.

The term "alkylsulfonyl" particularly includes methanesulfonyl, ethanesulfonyl, propylsulfonyl, 1-methylethylsulfonyl, butylsulfonyl, 2-methylpropylsulfonyl, 1-methylpropylsulfonyl, 1,1-dimethylethylsulfonyl, pentylsulfonyl, 3-methylbutylsulfonyl, 2-methylbutylsulfonyl, 2,2-dimethylpropylsulfonyl, 1-ethylpropylsulfonyl, 1,1-dimethylpropylsulfonyl, hexylsulfonyl, 4-methylpentylsulfonyl, 3-methylpentylsulfonyl, 2-methylpentylsulfonyl, 1-methylpentylsulfonyl, 3,3-dimethylbutylsulfonyl, 2,2-dimethylbutylsulfonyl, 1,1-dimethylbutylsulfonyl, 1,2-dimethylbutylsulfonyl, heptylsulfonyl, 1-methylhexylsulfonyl, 1-ethylpentylsulfonyl, octylsulfonyl, 1-methylheptylsulfonyl, 2-ethylhexylsulfonyl, nonylsulfonyl, decylsulfonyl, etc. Preferable one is alkylsulfonyl with 1 to 6 carbon atom(s), more preferably, straight chain or branched chain alkylsulfonyl with 1 to 4 carbon atom(s).

The term "alkylsulfinyl" particularly includes methylsulfinyl, ethylsulfinyl, propylsulfinyl, 1-methylethylsulfinyl, butylsulfinyl, 2-methylpropylsulfinyl, 1-methylpropylsulfinyl, 1,1-dimethylethylsulfinyl, pentylsulfinyl, 3-methylbutylsulfinyl, 2-methylbutylsulfinyl, 2,2-dimethylpropylsulfinyl, 1-ethylpropylsulfinyl, 1,1-dimethylpropylsulfinyl, hexylsulfinyl, 4-methylpentylsulfinyl, 3-methylpentylsulfinyl, 2-methylpentylsulfinyl, 1-methylpentylsulfinyl, 3,3-dimethylbutylsulfinyl, 2,2-dimethylbutylsulfinyl, 1,1-dimethylbutylsulfinyl, 1,2-dimethylbutylsulfinyl, heptylsulfinyl, 1-methylhexylsulfinyl, 1-ethylpentylsulfinyl, octylsulfinyl, 1-methylheptylsulfinyl, 2-ethylhexylsulfinyl, nonylsulfinyl, decylsulfinyl, etc. Preferable one is alkylsulfinyl with 1 to 6 carbon atom(s), more preferably, straight chain or branched chain alkylsulfinyl with 1 to 4 carbon atom(s).

The term "alkoxy" in "alkoxycarbonyl" includes the same as the alkoxy group as defined hereinbefore. Suitable examples of the alkoxycarbonyl are methoxycarbonyl, ethoxycarbonyl, propoxycarbonyl, 1-methylethoxycarbonyl, butoxycarbonyl, 2-methylpropoxycarbonyl, 1-methylpropoxycarbonyl, 1,1-dimethylethoxycarbonyl, pentoxycarbonyl, 3-methylbutoxycarbonyl, 2-methylbutoxycarbonyl, 2,2-dimethylpropoxycarbonyl, 1-ethylpropoxycarbonyl, 1,1-dimethylpropoxycarbonyl, hexyloxycarbonyl, 4-methylpentyloxycarbonyl, 3-methylpentyloxycarbonyl, 2-methylpentyloxycarbonyl, 1-methylpentyloxycarbonyl, 3,3-dimethylbutoxycarbonyl, 2,2-dimethylbutoxycarbonyl, 1,1-dimethylbutoxycarbonyl, 1,2-dimethylbutoxycarbonyl, heptyloxycarbonyl, 1-methylhexyloxycarbonyl, 1-ethylpentyloxycarbonyl, octyloxycarbonyl, 1-methylheptyloxycarbonyl, 2-ethylhexyloxycarbonyl, nonyloxycarbonyl, decyloxycarbonyl, etc. Preferable one is alkoxycarbonyl with 2 to 6 carbon atoms, more preferably, straight chain or branched chain alkoxycarbonyl with 2 to 4 carbon atoms.

The term "alkenyl" in "alkenyloxy", "alkenyloxycarbonyl", "alkenylcarbonyl", "alkenylcarbonyloxy", "alkenylsulfonyl" and "alkenylsulfinyl" includes the same as the alkenyl group as defined hereinbefore.

The term "alkenyloxy" particularly includes ethenyloxy, propenyloxy, 1-methylethenyloxy, butenyloxy, 2-methylpropenyloxy, 1-methylpropenyloxy, pentenyloxy, 3-methylbutenyloxy, 2-methylbutenyloxy, 1-ethylpropenyloxy, hexenyloxy, 4-methylpentenyloxy, 3-methylpentenyloxy, 2-methylpentenyloxy, 1-methylpentenyloxy, 3,3-dimethylbutenyloxy, 1,2-dimethylbutenyloxy, heptenyloxy, 1-methylhexenyloxy, 1-ethylpentenyloxy, octenyloxy, 1-methylheptenyloxy, 2-ethylhexenyloxy, nonenyloxy, decenyloxy, etc. Preferable one is alkenyloxy with 2 to 6 carbon atoms, more preferably, alkenyloxy with 2 to 5 carbon atoms.
The term "alkenylcarbonyl" particularly includes ethenylcarbonyl, propenylcarbonyl, 1-methylethenylcarbonyl, butenylcarbonyl, 2-methylpropenylcarbonyl, 1-methylpropenylcarbonyl, pentenylcarbonyl, 3-methylbutenylcarbonyl, 2-methylbutenylcarbonyl, 1-ethylpropenylcarbonyl, hexenylcarbonyl, 4-methylpentenylcarbonyl, 3-methylpentenylcarbonyl, 2-methylpentenylcarbonyl, 1-methylpentenylcarbonyl, 3,3-dimethylbutenylcarbonyl, 1,2-dimethylbutenylcarbonyl, heptenylcarbonyl, 1-methylhexenylcarbonyl, 1-ethylpentenylcarbonyl, octenylcarbonyl, 1-methylheptenylcarbonyl, 2-ethylhexenylcarbonyl, nonenylcarbonyl, decenylcarbonyl, etc. Preferable one is alkenylcarbonyl with 3 to 6 carbon atoms, more preferably, alkenylcarbonyl with 3 to 5 carbon atoms.

The term "alkenylcarbonyloxy" particularly includes those in which an oxygen atom binds to carbonyl of the above "alkenylcarbonyl". Preferable one is alkenylcarbonyloxy with 3 to 6 carbon atoms, more preferably, alkenylcarbonyloxy with 3 to 5 carbon atoms.

The term "alkenyloxycarbonyl" particularly includes ethenyloxycarbonyl, propenyloxycarbonyl, 1-methylethenyloxycarbonyl, butenyloxycarbonyl, 2-methylpropenyloxycarbonyl, 1-methylpropenyloxycarbonyl, pentenyloxycarbonyl, 3-methylbutenyloxycarbonyl, 2-methylbutenyloxycarbonyl, 1-ethylpropenyloxycarbonyl, hexenyloxycarbonyl, 4-methylpentenyloxycarbonyl, 3-methylpentenyloxycarbonyl, 2-methylpentenyloxycarbonyl, 1-methylpentenyloxycarbonyl, 3,3-dimethylbutenyloxycarbonyl, 1,2-dimethylbutenyloxycarbonyl, heptenyloxycarbonyl, 1-methylhexenyloxycarbonyl, 1-ethylpentenyloxycarbonyl, octenyloxycarbonyl, 1-methylheptenyloxycarbonyl, 2-ethylhexenyloxycarbonyl, nonenyloxycarbonyl, decenyloxycarbonyl, etc. Preferable one is alkenyloxycarbonyl with 3 to 6 carbon atoms, more preferably, alkenyloxycarbonyl with 3 to 5 carbon atoms.

The term "alkenylsulfonyl" particularly includes ethenylsulfonyl, propenylsulfonyl, 1-methylethenylsulfonyl, butenylsulfonyl, 2-methylpropenylsulfonyl, 1-methylpropenylsulfonyl, pentenylsulfonyl, 3-methylbutenylsulfonyl, 2-methylbutenylsulfonyl, 1-ethylpropenylsulfonyl, hexenylsulfonyl, 4-methylpentenylsulfonyl, 3-methylpentenylsulfonyl, 2-methylpentenylsulfonyl, 1-methylpentenylsulfonyl, 3,3-dimethylbutenylsulfonyl, 1,2-dimethylbutenylsulfonyl, heptenylsulfonyl, 1-methylhexenylsulfonyl, 1-ethylpentenylsulfonyl, octenylsulfonyl, 1-methylheptenylsulfonyl, 2-ethylhexenylsulfonyl, nonenylsulfonyl, decenylsulfonyl, etc. Preferable one is alkenylsulfonyl with 2 to 6 carbon atoms, more preferably, alkenylsulfonyl with 2 to 5 carbon atoms.

The term "alkenylsulfinyl" particularly includes ethenylsulfinyl, propenylsulfinyl, 1-methylethenylsulfinyl, butenylsulfinyl, 2-methylpropenylsulfinyl, 1-methylpropenylsulfinyl, pentenylsulfinyl, 3-methylbutenylsulfinyl, 2-methylbutenylsulfinyl, 1-ethylpropenylsulfinyl, hexenylsulfinyl, 4-methylpentenylsulfinyl, 3-methylpentenylsulfinyl, 2-methylpentenylsulfinyl, 1-methylpentenylsulfinyl, 3,3-dimethylbutenylsulfinyl, 1,2-dimethylbutenylsulfinyl, heptenylsulfinyl, 1-methylhexenylsulfinyl, 1-ethylpentenylsulfinyl, octenylsulfinyl, 1-methylheptenylsulfinyl, 2-ethylhexenylsulfinyl, nonenylsulfinyl, decenylsulfinyl, etc. Preferable one is alkenylsulfinyl with 2 to 6 carbon atoms, more preferably, alkenylsulfinyl with 2 to 5 carbon atoms.

The term "alkynyl" in "alkynyloxy", "alkynylcarbonyl", "alkynylcarbonyloxy", "alkynylsulfonyl", "alkynylsulfinyl" and "alkynyloxycarbonyl" includes the same as the alkynyl group as defined hereinbefore.

The term "alkynyloxy" particularly includes ethynyloxy, propynyloxy, butynyloxy, pentynyloxy, 3-methylbutynyloxy, hexynyloxy, 4-methylpentynyloxy, 3-methylpentynyloxy, 3,3-dimethylbutynyloxy, heptynyloxy, octynyloxy, 3-methylheptynyloxy, 3-ethylhexynyloxy, nonyloxy, decynyloxy, etc. Preferable one is alkynyloxy with 2 to 6 carbon atoms, more preferably, alkynyloxy with 2 to 5 carbon atoms.

The term "alkynylcarbonyl" particularly includes ethynylcarbonyl, propynylcarbonyl, butynylcarbonyl, pentynylcarbonyl, 3-methylbutynylcarbonyl, hexynylcarbonyl, 4-methylpentynylcarbonyl, 3-methylpentynylcarbonyl, 3,3-dimethylbutynylcarbonyl, heptynylcarbonyl, octynylcarbonyl, 3-methylheptynylcarbonyl, 3-ethylhexynylcarbonyl, nonylcarbonyl, decynylcarbonyl, etc. Preferable one is alkynylcarbonyl with 3 to 6 carbon atoms, more preferably, alkynylcarbonyl with 3 to 5 carbon atoms.

The term "alkynylcarbonyloxy" particularly includes those in which an oxygen atom binds to carbonyl in the above "alkynylcarbonyl". Preferable one is alkynylcarbonyloxy with 3 to 6 carbon atoms, more preferably, alkynylcarbonyloxy with 3 to 5 carbon atoms.

The term "alkynylsulfonyl" particularly includes ethynylsulfonyl, propynylsulfonyl, butynylsulfonyl, pentynylsulfonyl, 3-methylbutynylsulfonyl, hexynylsulfonyl, 4-methylpentynylsulfonyl, 3-methylpentynylsulfonyl, 3,3-dimethylbutynylsulfonyl, heptynylsulfonyl, octynylsulfonyl, 3-methylheptynylsulfonyl, 3-ethylhexynylsulfonyl, nonylsulfonyl, decynylsulfonyl, etc. Preferable one is alkynylsulfonyl with 2 to 6 carbon atoms, more preferably, alkynylsulfonyl with 2 to 5 carbon atoms.

The term "alkynylsulfinyl" particularly includes ethynylsulfinyl, propynylsulfinyl, butynylsulfinyl, pentynylsulfinyl, 3-methylbutynylsulfinyl, hexynylsulfinyl, 4-methylpentynylsulfinyl, 3-methylpentynylsulfinyl, 3,3-dimethylbutynylsulfinyl, heptynylsulfinyl, octynylsulfinyl, 3-methylheptynylsulfinyl, 3-ethylhexynylsulfinyl, nonylsulfinyl, decynylsulfinyl, etc. Preferable one is alkynylsulfinyl with 2 to 6 carbon atoms, more preferably, alkynylsulfinyl with 2 to 5 carbon atoms.

The term "alkynyloxycarbonyl" particularly includes ethynyloxycarbonyl, propynyloxycarbonyl, butynyloxycarbonyl, pentynyloxycarbonyl, 3-methylbutynyloxycarbonyl, hexynyloxycarbonyl, 4-methylpentynyloxycarbonyl, 3-methylpentynyloxycarbonyl, 3,3-dimethylbutynyloxycarbonyl, heptynyloxycarbonyl, octynyloxycarbonyl, 3-methylheptynyloxycarbonyl, 3-ethylhexynyloxycarbonyl, nonyloxycarbonyl, decynyloxycarbonyl, etc. Preferable one is alkynyloxycarbonyl with 3 to 6 carbon atoms, more preferably, alkynyloxycarbonyl with 3 to 5 carbon atoms.

The term "cycloalkyl" in "cycloalkylcarbonyl", "cycloalkylcarbonyloxy", "cycloalkylsulfonyl" and "cycloalkylsulfinyl" includes the same as the cycloalkyl as defined hereinbefore.

The term "cycloalkylcarbonyl" particularly includes cyclopropylcarbonyl, cyclobutylcarbonyl, cyclopentylcarbonyl, cyclohexylcarbonyl, cycloheptylcarbonyl or cyclooctylcarbonyl.

The term "cycloalkylcarbonyloxy" particularly includes those in which an oxygen atom binds to carbonyl of the above "cycloalkylcarbonyl". Suitable examples are cyclopropylcarbonyloxy, cyclobutylcarbonyloxy, cyclopentylcarbonyloxy, cyclohexylcarbonyloxy, cycloheptylcarbonyloxy or cyclooctylcarbonyloxy.

The term "cycloalkylsulfonyl" particularly includes cyclopropylsulfonyl, cyclobutylsulfonyl, cyclopentylsulfonyl, cyclohexylsulfonyl, cycloheptylsulfonyl or cyclooctylsulfonyl.

The term "cycloalkylsulfinyl" particularly includes cyclopropylsulfinyl, cyclobutylsulfinyl, cyclopentylsulfinyl, cyclohexylsulfinyl, cycloheptylsulfinyl or cyclooctylsulfinyl.

The term "cycloalkoxy" in "cycloalkoxycarbonyl" includes the same as the cycloalkoxy group as defined hereinbefore. Suitable examples are cyclopropyloxycarbonyl, cyclobutyloxycarbonyl, cyclopentyloxycarbonyl, cyclohexyloxycarbonyl, cycloheptyloxycarbonyl or cyclooctyloxycarbonyl.

The term "aryl" in "aryloxy", "arylcarbonyl", "aryloxycarbonyl", "arylcarbonyloxy", "arylsulfonyl" and "arylsulfinyl" includes the same as the aryl group as defined hereinbefore. Suitable examples of "aryloxy" are phenoxy, 1-naphthoxy or 2-naphthoxy. Suitable examples of "arylcarbonyl" are benzoyl, 1-naphthaloyl or 2-naphthaloyl. Particularly, "aryloxycarbonyl" includes phenoxycarbonyl, 1-naphthoxycarbonyl or 2-naphthoxycarbonyl. Suitable examples of "arylcarbonyloxy" are benzoyloxy, 1-naphthoyloxy or 2-naphthoyloxy. Particularly, "arylsulfonyl" includes phenylsulfonyl, 1-naphthylsulfonyl, 2-naphthylsulfonyl. Particularly, "arylsulfinyl" includes phenylsulfinyl, 1-naphthylsulfinyl, 2-naphthylsulfinyl.

The term "heteroaryl" in "heteroaryloxy", "heteroarylcarbonyl", "heteroaryloxycarbonyl", "heteroarylcarbonyloxy", "heteroarylsulfonyl" and "heteroarylsulfinyl" includes the same as the heteroaryl group as defined hereinbefore. Suitable examples of "heteroaryloxy" are pyrrolyloxy, pyridyloxy, pyrazinyloxy, pyrimidinyloxy, pyridazinyloxy, furyloxy, thienyloxy. Suitable examples of "heteroarylcarbonyl" are pyrrolylcarbonyl, pyridylcarbonyl, pyrazinylcarbonyl, pyrimidinylcarbonyl, pyridazinylcarbonyl, furylcarbonyl, thienylcarbonyl, etc. Suitable examples of "heteroaryloxycarbonyl" are pyrrolyloxycarbonyl, pyridyloxycarbonyl, pyrazinyloxycarbonyl, pyrimidinyloxycarbonyl, pyridazinyloxycarbonyl, furyloxycarbonyl, thienyloxycarbonyl.

Suitable examples of "heteroarylcarbonyloxy" are pyrrolylcarbonyloxy, pyridylcarbonyloxy, pyrazinylcarbonyloxy, pyrimidinylcarbonyloxy, pyridazinylcarbonyloxy, furylcarbonyloxy, thienylcarbonyloxy. Suitable examples of "heteroarylsulfonyl" are pyrrolylsulfonyl, pyridylsulfonyl, pyrazinylsulfonyl, pyrimidinylsulfonyl, pyridazinylsulfonyl, furylsulfonyl, thienylsulfonyl. Suitable examples of "heteroarylsulfinyl" are pyrrolylsulfinyl, pyridylsulfinyl, pyrazinylsulfinyl, pyrimidinylsulfinyl, pyridazinylsulfinyl, furylsulfinyl, thienylsulfinyl.

The substituents of the substituted alkyl, alkenyl and alkynyl herein include the following (a) to (c):
(a) halogen, hydroxyl, carboxy, haloalkyl, haloalkoxy, mercapto;
(b) alkoxy, alkylthio, alkylcarbonyl, alkylcarbonyloxy, alkylsulfonyl, alkylsulfinyl, alkoxycarbonyl, alkenyloxy, alkenylthio, alkenylcarbonyl, alkenylcarbonyloxy, alkenylsulfonyl, alkenylsulfinyl, alkenyloxycarbonyl, alkynyloxy, alkynylthio, alkynylcarbonyl, alkynylcarbonyloxy, alkynyloxycarbonyl, alkynylsulfonyl, alkynylsulfinyl,
   wherein each group may further be substituted by halogen, hydroxyl, alkoxy, carboxyl, alkoxycarbonyl, amino substituted by the same or different 1 or 2 alkyl(s), carbamoyl substituted by the same or different 1 or 2 alkyl(s), sulfamoyl substituted by the same or different 1 or 2 alkyl(s) or alkylsulfonyl;
(c) substituted or unsubstituted amino, substituted or unsubstituted carbamoyl, substituted or unsubstituted sulfamoyl, substituted or unsubstituted aryl, substituted or unsubstituted aryloxy, substituted or unsubstituted arylcarbonyl, substituted or unsubstituted arylcarbonyloxy, substituted or unsubstituted arylsulfonyl, substituted or unsubstituted arylsulfinyl, substituted or unsubstituted aryloxycarbonyl, substituted or unsubstituted heteroaryl, substituted or unsubstituted heteroaryloxy, substituted or unsubstituted heteroarylcarbonyl, substituted or unsubstituted heteroarylcarbonyloxy, substituted or unsubstituted heteroarylsulfonyl, substituted or unsubstituted heteroarylsulfinyl, substituted or unsubstituted heteroaryloxycarbonyl, substituted or unsubstituted cycloalkyl, substituted or unsubstituted cycloalkoxy, substituted or unsubstituted cycloalkylcarbonyl, substituted or unsubstituted cycloalkylcarbonyloxy, substituted or unsubstituted cycloalkylsulfonyl, substituted or unsubstituted cycloalkylsulfinyl, substituted or unsubstituted cycloalkoxycarbonyl, or substituted or unsubstituted saturated heterocycle, etc.;
said substituents being the same or different and being substituted on each group by 1 or more, preferably 1 to 5, more preferably 1 to 3 thereof.

The substituents of the substituted cycloalkyl, cycloalkoxy, cycloalkylcarbonyl, cycloalkylsulfonyl, cycloalkylsulfinyl, cycloalkylcarbonyloxy, cycloalkoxycarbonyl or saturated heterocycle herein include the following (d) to (f):
(d) halogen, hydroxyl, carboxy, mercapto, haloalkyl, haloalkoxy;
(e) alkyl, alkenyl, alkynyl, alkoxy, alkylthio, alkylcarbonyl, alkylcarbonyloxy, alkylsulfonyl, alkylsulfinyl, alkoxycarbonyl,
   wherein each group may further be substituted by halogen, hydroxyl, alkoxy, carboxy, alkoxycarbonyl, amino substituted by the same or different 1 or 2 alkyl(s), carbamoyl substituted by the same or different 1 or 2 alkyl(s), sulfamoyl substituted by the same or different 1 or 2 alkyl(s) or alkylsulfonyl;
(f) substituted or unsubstituted aryl, substituted or unsubstituted heteroaryl, substituted or unsubstituted amino, substituted or unsubstituted carbamoyl, substituted or unsubstituted sulfamoyl, etc.;
said substituents being the same or different and being substituted on each group by 1 or more, preferably 1 to 5, more preferably 1 to 3 thereof.

The substituents of the substituted aryl, heteroaryl, aryloxy, arylcarbonyl, arylcarbonyloxy, arylsulfonyl, arylsulfinyl, aryloxycarbonyl, heteroaryloxy, heteroarylcarbonyl, heteroarylcarbonyloxy, heteroarylsulfonyl, heteroarylsulfinyl or heteroaryloxycarbonyl herein include the following (g) to (i):
(g) halogen, hydroxyl, carboxy, mercapto, cyano, nitro, haloalkyl, haloalkoxy;
(h) alkyl, alkoxy, alkylthio, alkylcarbonyl, alkoxycarbonyl, alkylcarbonyloxy, alkylsulfonyl, alkylsulfinyl, alkenyl, alkynyl, cycloalkyl, saturated heterocycle,
   wherein each group may further be substituted by halogen, hydroxyl, alkyl, alkoxy, carboxyl, alkoxycarbonyl, amino substituted by the same or different 1 or 2 alkyl(s), carbamoyl substituted by the same or different 1 or 2 alkyl(s), sulfamoyl substituted by the same or different 1 or 2 alkyl(s) or alkylsulfonyl;
(i) substituted or unsubstituted amino, substituted or unsubstituted carbamoyl, substituted or unsubstituted sulfamoyl, etc.;
said substituents being the same or different and being substituted on each group by 1 or more, preferably 1 to 5, more preferably 1 to 3 thereof.

The substituents in the substituted or unsubstituted "amino", substituted or unsubstituted "carbamoyl" and substituted or unsubstituted "sulfamoyl" include the following (j), (k) and (l):
(j) alkyl, alkenyl, alkynyl, alkylcarbonyl, alkylcarbonyloxy, alkoxycarbonyl, alkylsulfonyl, alkylsulfinyl, alkenylcarbonyl, alkenylcarbonyloxy, alkenyloxycarbonyl, alkenylsulfonyl, alkenylsulfinyl, alkynylcarbonyl, alkynylcarbonyloxy, alkynyloxycarbonyl, alkynylsulfonyl, alkynylsulfinyl, cycloalkyl, cycloalkylcarbonyl, cycloalkylcarbonyloxy, cycloalkoxycarbonyl, cycloalkylsulfonyl, cycloalkylsulfinyl, saturated heterocycle,
   wherein each group may further be substituted by halogen, hydroxyl, alkoxy, carboxy, alkoxycarbonyl, amino substituted by the same or different 1 or 2 alkyl(s), carbamoyl substituted by the same or different 1 or 2 alkyl(s), sulfamoyl substituted by the same or different 1 or 2 alkyl(s) or alkylsulfonyl;
(k) aryl, arylcarbonyl, arylcarbonyloxy, aryloxycarbonyl, arylsulfonyl, arylsulfinyl, heteroaryl, heteroarylcarbonyl, heteroarylcarbonyloxy, heteroaryloxycarbonyl, heteroarylsulfonyl, heteroarylsulfinyl,
   wherein each group may further be substituted by halogen, hydroxyl, alkyl, alkoxy, carboxy, alkoxycarbonyl, amino substituted by the same or different 1 or 2 alkyl(s), carbamoyl substituted by the same or different 1 or 2 alkyl(s), sulfamoyl substituted by the same or different 1 or 2 alkyl(s) or alkylsulfonyl;
(l) when 2 substituents of amino, carbamoyl and sulfamoyl are combined together with nitrogen atom to form 4- to 7-membered saturated nitrogen-containing heterocycle with 1 to 4 heteroatom(s) selected from 1 to 2 nitrogen(s), 0 to 1 oxygen and 0 to 1 sulfur,
   wherein the saturated nitrogen-containing heterocycle may further be substituted on any carbon atoms or nitrogen atoms by halogen, hydroxyl, alkoxy, carboxy, alkoxycarbonyl, amino substituted by the same or different 1 or 2 alkyl(s), carbamoyl substituted by the same or different 1 or 2 alkyl(s), sulfamoyl substituted by the same or different 1 or 2 alkyl(s) or alkylsulfonyl, where the substituent may be kept in chemically stable state, etc.;
said substituents being substituted by 1 or 2 substituent(s) where those may be kept in chemically stable state.

The substituent of the substituted alkylene herein includes halogen, hydroxyl, alkoxy, substituted or unsubstituted aryl or substituted or unsubstituted heteroaryl, etc., which may be, the same or different, substituted by 1 or more, preferably 1 to 5, more preferably 1 to 3.

The term "4- to 7-membered saturated nitrogen-containing heterocycle" as used herein includes 4- to 7-membered saturated nitrogen-containing heterocycle containing 1 to 3 heteroatom(s) selected from 1 to 3 nitrogen(s), 0 to 1 oxygen and 0 to 1 sulfur, and the substituent may bind on any binding positions without any limitation where it may be kept in chemically stable state. Suitable examples are azetidine, pyrrolidine, piperidine, piperazine, morpholine, thiomorpholine, thiomorpholin-1-oxide, thiomorpholine-1,1-dioxide, perhydroazepine, imidazolidine, oxazolidine, etc.

The 3- to 7-membered saturated carbocycle formed by combining R³ and R⁵ together with the adjacent carbon atom includes cyclopropane, cyclobutane, cyclopentane, cyclohexane or cycloheptane.

The 3- to 7-membered saturated heterocycle formed by combining R³ and R⁵ together with the adjacent carbon atom includes 3- to 7-membered, preferably, 4- to 7-membered saturated heterocycle containing 1 to 2 heteroatom(s) selected from 1 to 2 nitrogen(s), 0 to 1 oxygen and 0 to 1 sulfur. Suitable examples are azetidine, pyrrolidine, piperidine, piperazine, morpholine, thiomorpholine, thiomorpholin-1-oxide, thiomorpholine-1,1-dioxide, tetrahydrofuran, tetrahydropyrane, etc.

The substituents of the substituted saturated carbocycle and saturated heterocycle include those described in the above (d) to (f).

In formula (1), R² is preferably alkyl with 1 to 4 carbon atom(s), alkylcarbonyloxyalkyl with 3 to 8 carbon atoms, arylcarbonyloxyalkyl, heteroarylcarbonyloxyalkyl, or alkyl substituted by substituted or unsubstituted amino.

The alkyl substituted by substituted or unsubstituted amino preferably includes alkyl substituted by dialkylaminoalkyl, morpholino, 1-piperidinyl, piperazino or 1-pyrrolidinyl.

The alkylcarbonyloxyalkyl particularly includes acetoxymethyl, 1-acetoxyethyl, etc. The arylcarbonyloxyalkyl particularly includes benzoyloxymethyl, etc. The alkyl substituted by substituted or unsubstituted amino particularly includes dialkylaminoalkyl such as dimethylaminobutyl, 4-morpholinobutyl, etc. More preferably, R² is methyl.

In formula (1) wherein X is NR⁶, R⁶ is preferably hydrogen or alkyl with 1 to 3 carbon atom(s), more preferably, hydrogen or methyl. X is preferably oxygen or a single bond.

In formula (1), R¹ is preferably substituted or unsubstituted straight chain or branched chain alkyl with 1 to 6 carbon atom(s), particularly, substituted or unsubstituted methyl, ethyl, propyl, butyl, pentyl, 1-methylethyl, 1-methylpropyl, 2-methylbutyl, etc. More preferable one is straight-chain alkyl with 1 to 4 carbon atom(s).

In case that R¹ is substituted alkyl, the substituent of the alkyl preferably includes fluorine, hydroxyl, straight chain or branched chain alkoxy with 1 to 4 carbon atom(s), or straight chain or branched chain alkylthio with 1 to 4 carbon atom(s), etc. More preferably, the substituent includes hydroxyl, or straight chain or branched chain alkoxy with 1 to 3 carbon atom(s).

In formula (1), Z preferably includes substituted or unsubstituted alkylene with 1 to 6, preferably 2 to 6, carbon atom(s), etc., particularly, ethylene, propylene, butylene, etc. The substituent of the substituted alkylene in Z includes halogen, hydroxyl, alkoxy with 1 to 6 carbon atom(s), substituted or unsubstituted aryl, or substituted or unsubstituted heteroaryl, wherein substituents of the aryl and the heteroaryl include halogen, hydroxyl, alkyl with 1 to 6 carbon atom(s), alkoxy with 1 to 6 carbon atom(s), haloalkyl with 1 to 6 carbon atom(s), haloalkoxy with 1 to 6 carbon atom(s), and amino optionally substituted by the same or different 1 or 2 alkyl(s) with 1 to 6 carbon atom(s), which may be, the same or different, substituted on each group by 1 to 4 thereof.

In formula (1), R³ and R⁴ are preferably hydrogen or unsubstituted alkyl with 1 to 3 carbon atom(s), particularly, hydrogen, methyl or ethyl. R⁵ is preferably hydrogen or substituted or unsubstituted straight chain or branched chain alkyl with 1 to 6 carbon atom(s), particularly, hydrogen, or substituted or unsubstituted methyl, ethyl, propyl, butyl, pentyl, 1-methylethyl, 1-methylpropyl, 2-methylbutyl, etc. More preferable one is alkyl with 1 to 4 carbon atom(s) optionally substituted by alkoxycarbonyl with 2 to 5 carbon atoms, carboxy, hydroxyl, amino optionally substituted by the same or different 1 or 2 alkyl(s) with 1 to 6 carbon atom(s), carbamoyl and 6- to 10-membered aryl, wherein the aryl may be substituted by 1 or more of the same or different substituent(s) independently selected from halogen; hydroxyl; alkyl with 1 to 6 carbon atom(s) or alkoxy with 1 to 6 carbon atom(s) which may be substituted by amino optionally substituted by hydroxyl, alkoxy with 1 to 6 carbon atom(s) or the same or different 1 or 2 alkyl(s) with 1 to 6 carbon atom(s); haloalkyl with 1 to 6 carbon atom(s); haloalkoxy with 1 to 6 carbon atom(s); and amino optionally substituted by the same or different 1 or 2 alkyl(s) with 1 to 6 carbon atom(s).

The adenine compounds of the present invention are intended to include all tautomers, geometric isomers or stereoisomers, and optionally, a mixture thereof depending on the kinds of substituents.
In other words, in case that one or more asymmetric carbon atom(s) may exist in the compound of the formula (1), diastereomers and enantiomers may also exist, and the present invention includes the diastereomers, the enantiomers, and mixtures and isolated forms thereof.

Additionally, the adenine compound of the formula (1) and a tautomer thereof are chemically equivalent, and the adenine compound of the present invention also includes the tautomer thereof. Particularly, the tautomer is in the form of hydroxy of the formula (1'): 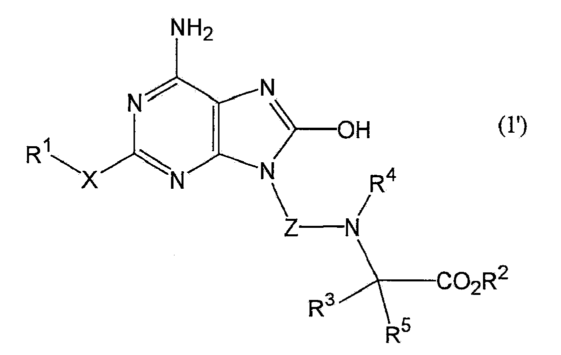 wherein R¹, R², R³, R⁴, R⁵, X and Z are the same as defined above.

A pharmaceutically acceptable salt includes acid addition salt and base addition salt. For example, the acid addition salt includes an inorganic acid salt such as hydrochloride, hydrobromide, sulfate, hydroiodide, nitrate, phosphate, and an organic acid salt such as citrate, oxalate, acetate, formate, propionate, benzoate, trifluoroacetate, fumarate, maleate, succinate, tartrate, lactate, pyruvate, methanesulfonate, benzenesulfonate, para-toluenesulfonate, and the base addition salt includes an inorganic base salt such as sodium salt, potassium salt, calcium salt, magnesium salt, ammonium salt, and an organic base salt such as triethyl ammonium salt, triethanol ammonium salt, pyridinium salt, diisopropyl ammonium salt, and additionally, amino acid salt such as basic or acidic amino acid including arginine, aspartic acid and glutamic acid. The compound of the formula (1) may be a hydrate, or a solvate such as ethanolate.

The compound of the general formula (1) may be prepared by the following methods. Starting compounds which are not described below may be prepared according to the following methods or known methods or those similar thereto.

### Preparation Method 1

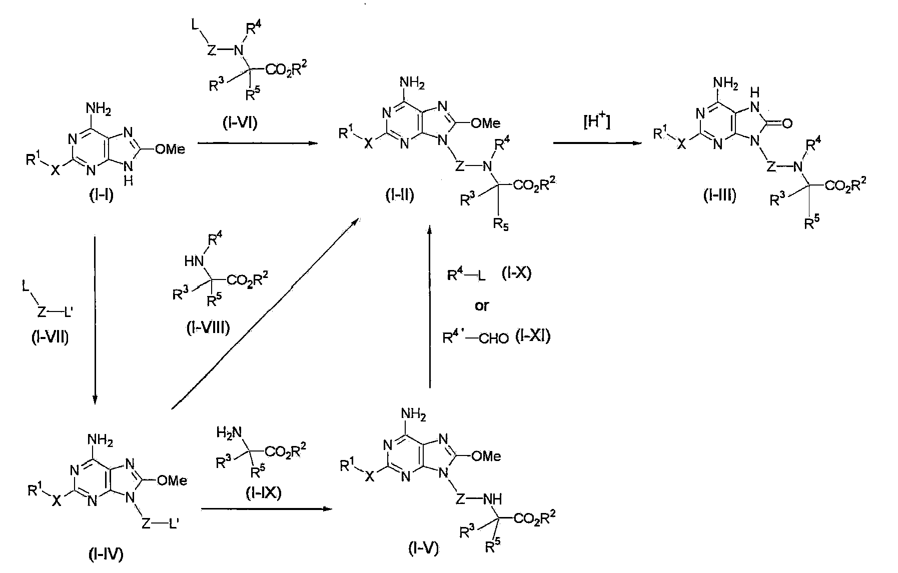

In the above Scheme, R¹, R², R³, R⁴, R⁵, X and Z are the same as defined above, L and L' are leaving groups which may be the same or different each other, and R^{4'} is combined together with methylene to represent R⁴.
In the Scheme, a protection or deprotection technique may be applied to N atoms, if necessary. A preferable protective group, a method for protection and deprotection are particularly described in "Protective Groups in Organic Synthesis 2nd Edition (John Wiley & Sons, Inc.; 1990)" etc.

Compound (I-I) may be reacted with compound (I-VI) in the presence of a base to give compound (I-II). For example, the base which may be used therein includes alkali metal carbonate such as sodium carbonate or potassium carbonate, alkaline earth metal carbonate such as calcium carbonate, metal hydroxide such as sodium hydroxide or potassium hydroxide, metal hydride such as sodium hydride, or metal alkoxide such as potassium t-butoxide, etc. For example, the solvent which may be used therein includes aprotic solvent such as dimethylformamide, dimethylsulfoxide or acetonitrile, halogenated hydrocarbon solvent such as carbon tetrachloride, chloroform or methylene chloride, ether solvent such as diethyl ether, tetrahydrofuran or 1,4-dioxane, etc. For example, the reaction temperature is selected from the range of about 0°C to around a boiling point of the solvent.

In the synthesis of compound (I-II) from compound (I-I), compound (I-I) may be also reacted with compound (I-VII) under the same basic condition as described above to give compound (I-IV), followed by reacting with compound (I-VIII) under the same condition to give compound (I-II).

In the synthesis of compound (I-II) from compound (I-IV), compound (I-IV) may be also reacted with compound (I-IX) under the same condition as described above to give compound (I-V), followed by reacting with compound (I-X) under the same condition to give compound (I-II).

In the synthesis of compound (I-II) from compound (I-V), compound (I-V) may be also reacted with aldehyde compound (I-XI) using a reductant such as sodium borohydride (NaBH₄) in a solvent such as methanol to give compound (I-II).

Compound (I-II) may be treated under an acidic condition to give compound (I-III). For example, the acid used in the acid-treatment includes an inorganic acid such as hydrochloric acid, hydrobromic acid or sulfuric acid, or an organic acid such as trifluoroacetic acid, etc. For example, the solvent which may be used therein includes water, or a mixture of water and an organic solvent. The organic solvent includes ether solvent such as diethyl ether or tetrahydrofuran, aprotic solvent such as dimethylformamide or acetonitrile, or alcoholic solvent such as methanol or ethanol, etc. The reaction temperature is, for example, selected from the range of room temperature to around a boiling point of the solvent.

Compound (I-VI) may be prepared by the following methods. 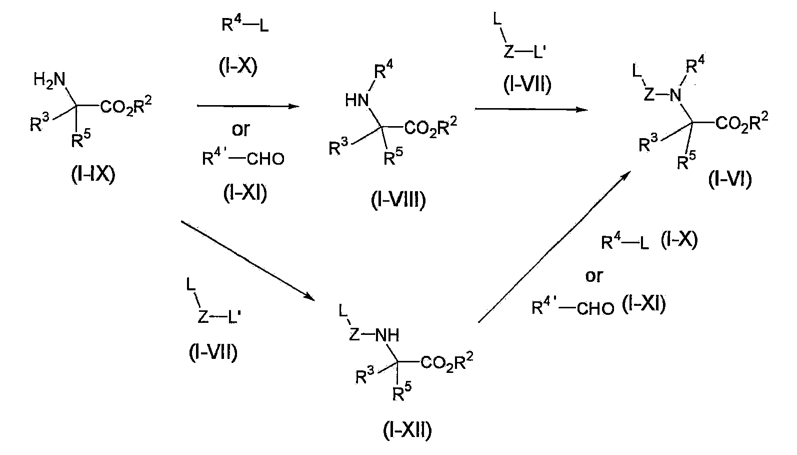 In the above Scheme, L, L', R², R³, R⁴, R^{4'}, R⁵ and Z are the same as defined above.
Compound (I-IX) may be reacted with compound (I-X) in the presence of a base to give compound (I-VIII).

Compound (I-VIII) may be also obtained by reacting with compound (I-XI) under the same reduction condition as described above.

Subsequently, compound (I-VIII) may be reacted with compound (I-VII) in the presence of the same base as described above to give compound (I-VI).

In the synthesis of compound (I-VI) from compound (I-IX), compound (I-IX) may be also reacted with compound (I-VII) under the same condition as described above to give compound (I-XII), followed by reacting with compound (I-X) or compound (I-XI) under the same condition as described above to give compound (I-VI).

Compound (I-I) may be prepared by the following methods. 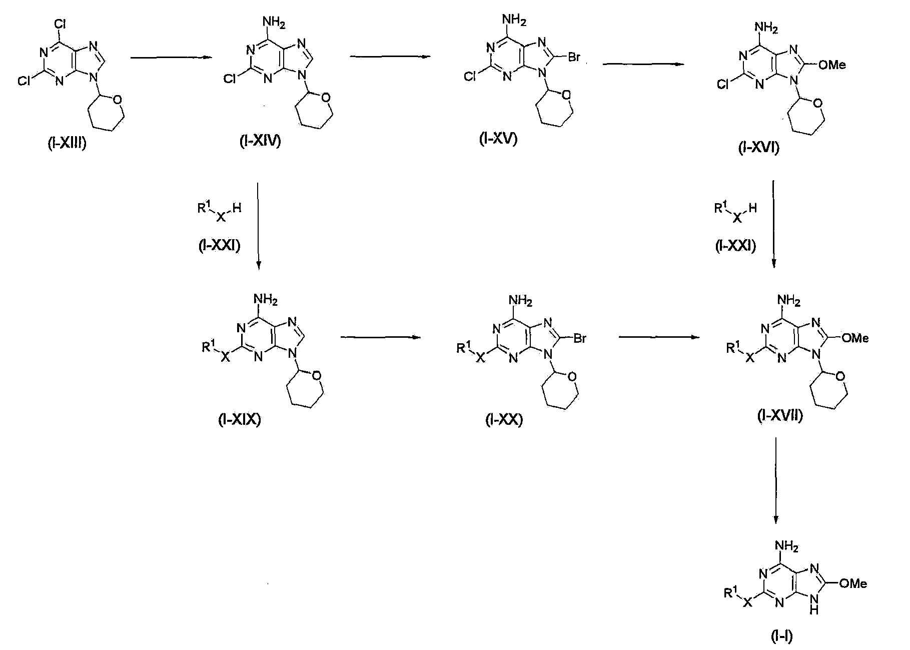 In the above Scheme, R¹ and X are the same as defined above.

Compound (I-XIII) may be reacted with ammonia in aqueous solution, organic solvent or a mixture of water and organic solvent to give compound (I-XIV).

For example, the organic solvent includes alcoholic solvent such as methanol, ethanol, propanol or butanol, ether solvent such as tetrahydrofuran, 1,4-dioxane or diglyme, aprotic solvent such as acetonitrile, etc. For example, the reaction temperature is selected from the range of room temperature to 200°C. A reaction container such as autoclave may be used in the reaction.

Compound (I-XIV) may be brominated to give compound (I-XV). For example, a brominating agent which may be used therein includes bromine, hydrobromic acid perbromide or N-bromosuccinimide, etc., and for example, a reaction auxiliary such as sodium acetate may be added to the reaction. For example, the solvent which may be used therein includes halogenated hydrocarbon solvent such as carbon tetrachloride, methylene chloride or dichloroethane, ether solvent such as diethyl ether, acetic acid, or carbon disulfide, etc. For example, the reaction temperature is selected from the range of about 0°C to around a boiling point of the solvent.

Compound (I-XV) may be reacted with sodium methoxide to give compound (I-XVI).

For example, the organic solvent which may be used therein includes ether solvent such as diethyl ether, tetrahydrofuran or 1,4-dioxane, aprotic solvent such as dimethylformamide, or alcoholic solvent such as methanol, etc. For example, the reaction temperature is selected from the range of room temperature to around a boiling point of the solvent.

Compound (I-XV) may be also treated in an aqueous alkaline solution containing methanol to give compound (I-XVI).

The aqueous alkaline solution which may be used therein includes an aqueous solution of alkali metal hydroxide such as sodium hydroxide or potassium hydroxide. For example, the reaction temperature is selected from the range of room temperature to around a boiling point of the solvent.

Compound (I-XVI) may be reacted with compound (I-XXI) to give compound (I-XVII).

The reaction is carried out in the presence or absence of a base in case that X is NR⁶ wherein R⁶ is hydrogen or alkyl. For example, the base which may be used therein includes alkali metal carbonate such as sodium carbonate or potassium carbonate, alkaline earth metal carbonate such as calcium carbonate, metal hydroxide such as sodium hydroxide or potassium hydroxide, or an organic base such as triethylamine, diisopropylethylamine or 4-dimethylaminopyridine, etc. For example, the solvent which may be used therein includes ether solvent such as tetrahydrofuran, 1,4-dioxane or diglyme, alcoholic solvent such as propanol or butanol, or aprotic solvent such as dimethylformamide, or the reaction may be carried out in the absence of solvent. For example, the reaction temperature is selected from the range of about 50°C to 200°C.

The reaction is carried out in the presence of a base in case that X is oxygen or sulfur. For example, the base which may be used therein includes alkaline metal such as sodium or potassium, or alkaline metal hydride such as sodium hydride. For example, the solvent which may be used therein includes ether solvent such as tetrahydrofuran, 1,4-dioxane or diglyme, or aprotic solvent such as dimethylformamide or dimethylsulfoxide, or the reaction may be carried out in the absence of solvent. For example, the reaction temperature is selected from the range of about 50°C to 200°C.

The reaction may be carried out by oxidizing the corresponding intermediate for preparation wherein X is sulfur with oxone^{™} or m-chloroperbenzoic acid (mCPBA) in case that X is SO₂.

Alternatively, in the preparation step from compound (I-XIV) to compound (I-XVII), compound (V-XIX) may be synthesized in the similar manner to the above to give compound (I-XX), followed by obtaining compound (I-XVII).

Compound (I-XVII) may be treated with trifluoroacetic acid in an organic solvent such as methanol to give compound (I-I).

For example, the acid which may be used therein includes an inorganic acid such as hydrochloric acid, hydrobromic acid or sulfuric acid, or an organic acid such as trifluoroacetic acid. For example, the solvent which may be used therein includes water, or a mixture of water and an organic solvent. The organic solvent includes ether solvent such as diethyl ether or tetrahydrofuran, aprotic solvent such as dimethylformamide or acetonitrile, or alcoholic solvent such as methanol or ethanol. For example, the reaction temperature is selected from the range of room temperature to around a boiling point of the solvent.

Preparation Method 2 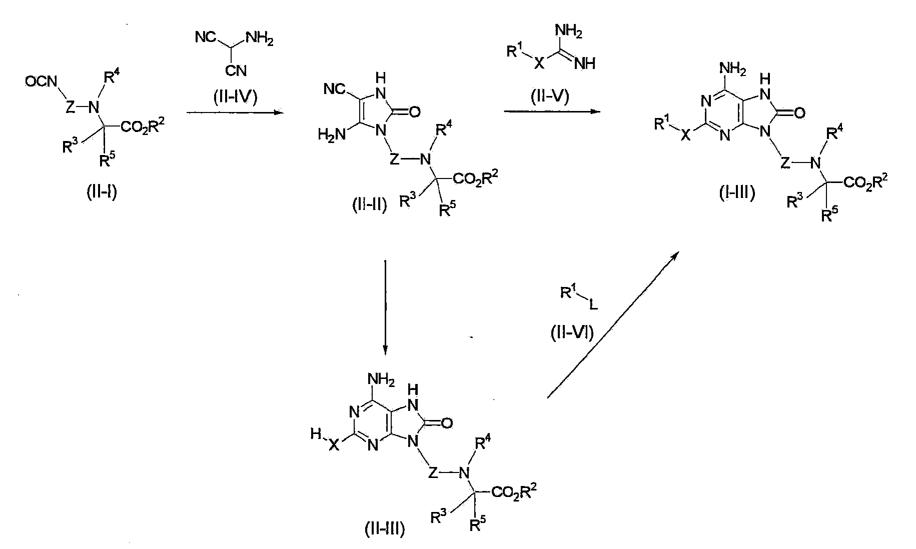 In the above Scheme, L is a leaving group, and R¹, R², R³, R⁴, R⁵, X and Z are the same as defined above.

Compound (II-I) may be reacted with compound (II-IV) in the presence of a base to give compound (II-II).

For example, the base which may be used therein includes alkali metal carbonate such as sodium carbonate or potassium carbonate, alkaline earth metal carbonate such as calcium carbonate, metal hydroxide such as sodium hydroxide or potassium hydroxide, an organic base such as triethylamine, diisopropylethylamine, pyridine or 4-dimethylaminopyridine, or metal alkoxide such as sodium methoxide. For example, the solvent which may be used therein includes halogenated hydrocarbon solvent such as methylene chloride, ether solvent such as diethyl ether, tetrahydrofuran or 1,4-dioxane, alcoholic solvent such as methanol or ethanol, or aprotic solvent such as dimethylformamide, dimethylsulfoxide or acetonitrile. For example, the reaction temperature is selected from the range of about 0°C to around a boiling point of the solvent.

Compound (II-II) may be reacted with compound (II-V) in the presence or absence of a base to give compound (I-III).

For example, the base which may be used therein includes an inorganic base including alkali metal carbonate such as sodium carbonate or potassium carbonate, alkaline earth metal carbonate such as calcium carbonate, or metal hydroxide such as sodium hydroxide or potassium hydroxide, an organic base such as triethylamine, diisopropylethylamine, pyridine or 4-dimethylaminopyridine, or metal alkoxide such as sodium methoxide. For example, the solvent which may be used therein includes ether solvent such as tetrahydrofuran, 1,4-dioxane or diglyme, alcoholic solvent such as methanol or ethanol, or aprotic solvent such as toluene, dimethylformamide or dimethylsulfoxide, or the reaction may be carried out in the absence of solvent. For example, the reaction temperature is selected from the range of room temperature to around a boiling point of the solvent.

Alternatively, in the preparation step from compound (II-II) to compound (I-III), compound (I-III) may also be obtained through synthesis of compound (II-III).

In case that X is NR⁶ wherein R⁶ is hydrogen or alkyl group, compound (II-II) may be reacted with guanidine in the presence or absence of a base to give compound (II-III).

In case that X is oxygen, compound (II-II) may be reacted with urea in the presence or absence of a base to give compound (II-III). For example, the base which may be used therein includes alkali metal carbonate such as sodium carbonate or potassium carbonate, alkaline earth metal carbonate such as calcium carbonate, metal hydroxide such as sodium hydroxide or potassium hydroxide, an organic base such as triethylamine, diisopropylethylamine, pyridine or 4-dimethylaminopyridine, or metal alkoxide such as sodium methoxide. For example, the solvent which may be used therein includes ether solvent such as tetrahydrofuran, 1,4-dioxane or diglyme, alcoholic solvent such as methanol or ethanol, or aprotic solvent such as toluene, dimethylformamide or dimethylsulfoxide, or the reaction may be carried out in the absence of solvent. For example, the reaction temperature is selected from the range of room temperature to around a boiling point of the solvent.

In case that X is sulfur, compound (II-II) may be reacted with benzoyl isocyanate in the presence or absence of a base, followed by carrying out a cyclization reaction to give compound (II-III).

In the reaction with benzoyl isocyanate, the base which may be used therein includes, for example, alkali metal carbonate such as sodium carbonate or potassium carbonate, alkaline earth metal carbonate such as calcium carbonate, or organic base such as triethylamine, diisopropylethylamine, pyridine or 4-dimethylaminopyridine, etc. For example, the solvent which may be used therein includes halogenated hydrocarbon solvent such as methylene chloride, ether solvent such as tetrahydrofuran or 1,4-dioxane, or aprotic solvent such as dimethylformamide or dimethylsulfoxide, etc. For example, the reaction temperature is selected from the range of about 0°C to around a boiling point of the solvent.

In the cyclization reaction, the base which may be used therein includes, for example, alkali metal hydroxide such as sodium hydroxide or potassium hydroxide, or metal alkoxide such as sodium methoxide or potassium t-butoxide, etc. For example, the solvent which may be used therein includes ether solvent such as tetrahydrofuran, alcoholic solvent such as ethanol or 2-propanol, or aprotic solvent such as dimethylformamide or dimethylsulfoxide, etc. For example, the reaction temperature is selected from the range of room temperature to around a boiling point of the solvent.

Compound (II-III) may be reacted with compound (II-VI) in the presence of the same base as described above to give compound (I-III).

The adenine compounds, intermediates or starting compounds thereof with any functional groups in the present invention may be optionally subjected to homologation reaction, substituent introduction reaction or functional group transformation reaction, etc. in an appropriate step, or more specifically, in any halfway step of each preparation method described in the above Preparation Method 1 or 2 according to a conventional method known to those skilled in the art. For these reactions, a method described in "Jikken-Kagaku-Koza (edited by the Chemical Society of Japan, Maruzen)", or "Comprehensive Organic Transformation, Author: R. C. Larock, (VCH Publishers, Inc, 1989)", etc. may be used. The homologation reaction includes, for example, a method wherein ester is converted into hydroxymethyl using a reducing agent such as lithium aluminum hydride, followed by introducing a leaving group to introduce cyano, etc. The functional group transformation reaction includes, for example, acylation or sulfonylation reaction using acid halide, sulfonyl halide, etc., a reaction using alkylating agent such as halogenated alkyl, hydrolysis reaction, carbon-carbon bond formation reaction such as Friedel-Crafts reaction or Wittig reaction, oxidation or reduction reaction, etc.

When the compound of the present invention or an intermediate thereof contains a functional group such as amino, carboxy, hydroxyl or oxo in the present invention, a protection or deprotection technique may optionally be applied. A preferable protective group, a method for protection and deprotection are specifically described in "Protective Groups in Organic Synthesis 2nd Edition (John Wiley & Sons, Inc.; 1990)", etc.

The compound of the formula (1) or an intermediate for preparing the same of the present invention may be purified by a method known to those skilled in the art. For example, it may be purified by column chromatography (e.g., silica gel column chromatography, or ionexchange column chromatography), or recrystallization, etc. The solvent which may be used in the recrystallization includes, for example, alcoholic solvents such as methanol, ethanol or 2-propanol, ether solvents such as diethyl ether, ester solvents such as ethyl acetate, aromatic hydrocarbon solvents such as benzene or toluene, ketone solvents such as acetone, hydrocarbon solvents such as hexane, aprotic solvents such as dimethylformamide or acetonitrile, water, or a mixture thereof, etc. Other purification methods include a method described in Jikken-Kagaku-Koza (edited by the Chemical Society of Japan, Maruzen), vol. 1, etc.

The compound of the formula (1) with 1 or more asymmetric center(s) of the present invention may be prepared by using a starting material with asymmetric centers or introducing asymmetric centers in any half way steps according to a conventional method. For example, enantiomers may be obtained by using optically active starting materials or carrying out optical resolution in an appropriate step of the preparation. For example, the optical resolution may be carried out by a diastereomeric method wherein the compound of the formula (1) or an intermediate thereof is reacted with an optically active acid (e.g., monocarboxylic acid such as mandelic acid, N-benzyloxyalanine or lactic acid, dicarboxylic acid such as tartaric acid, o-diisopropylidene tartaric acid or malic acid, or sulfonic acid such as camphorsulfonic acid or bromocamphorsulfonic acid) to form a salt thereof in an inactive solvent (e.g., alcoholic solvent such as methanol, ethanol or 2-propanol, ether solvent such as diethyl ether, ester solvent such as ethyl acetate, hydrocarbon solvent such as toluene, or aprotic solvent such as acetonitrile, and a mixture thereof).

The optical resolution may be also carried out by reacting the compound of the formula (1) or an intermediate thereof having an acidic functional group such as carboxy with an optically active amine (e.g., organic amine such as α-phenethylamine, quinine, quinidine, cinchonidine, cinchonine, strychnine) to form a salt thereof.

A temperature for forming the salt is selected from the range of room temperature to a boiling point of the solvent. In order to improve an optical purity, it is desirable to raise the temperature up to around a boiling point of the solvent. The precipitated salt may be cooled in filtration to improve its yield as necessary. The usage of an optically active acid or amine is properly in the range of about 0.5 to about 2.0 equivalents, preferably around 1 equivalent, to the substrate. The crystal may be also, as necessary, recrystallized in an inactive solvent (e.g., alcoholic solvent such as methanol, ethanol, 2-propanol, ether solvent such as diethyl ether, ester solvent such as ethyl acetate, hydrocarbon solvent such as toluene, aprotic solvent such as acetonitrile, and a mixture thereof) to give an optically active salt in high purity. The optically resolved salt may be also, as necessary, treated with acid or base in a conventional manner to give in a free form.

The adenine compound, or a pharmaceutically acceptable salt thereof of the present invention activates toll-like receptor (TLR), specifically TLR7, and is useful as an immune-regulating agent and a therapeutic or preventive agent for diseases such as diseases associated with abnormality of immune response (e.g., autoimmune diseases and allergic diseases), various infectious diseases wherein an immune response is desired to be activated or cancer. For example, the adenine compound or a pharmaceutically acceptable salt thereof of the present invention is useful as a therapeutic or preventive agent for diseases including the following (1) to (8).

(1) Respiratory affections, including intermittent or persistent asthma of every severity (e.g., bronchial asthma, allergic asthma, intrinsic asthma, extrinsic asthma, exercise-induced asthma, asthma induced by drug (e.g., NSAID such as aspirin and indometacin), dust-induced asthma, and reactive airway diseases caused by other factors); chronic obstructive lung disease (COPD); bronchitis (e.g., infectious bronchitis, eosinophilic bronchitis); emphysema; bronchiectasis; cystic fibrosis; sarcoidosis; farmer's lung and related diseases thereof; hypersensitivity pneumonitis; lung fibrosis (e.g., cryptogenic fibrosing alveolitis, idiopathic interstitial pneumonias, and fibrosis caused by antineoplastic therapy, chronic infectious diseases including tuberculosis bacterial, aspergillus or other fungal infectious diseases, etc.); complication by lung transplantation; vascular and thrombotic pulmonary disease and pulmonary hypertension; antitussive including treatment of chronic cough and iatrogenic cough associated with inflammation or secretion of airway; acute or chronic rhinitis including rhinitis medicamentosa or vasomotor rhinitis; perennial or seasonal allergic rhinitis including rhinitis nervosa (hay fever); nasal polyposis; acute virus infection including common cold disease and infectious diseases by respiratory syncytium virus, influenza, coronavirus (including SARS) and adenovirus;

(2) Cutaneous diseases, including psoriasis, atopic dermatitis, contact dermatitis and other eczematous dermatoses, and delayed-type hypersensitivity reaction; phyto- and photodermatitis; seborrheic dermatitis, herpetiformis dermatitis; lichen planus, lichen sclerosis, lichen sclerosus et atrophicus, pyoderma gangrenosum, dermal sarcoidosis, discoid lupus erythematosus, pemphigus, pemphigoid, epidermolysis bullosa, urticaria, angioedema, vasculitides, toxic erythema, cutaneous eosinophilia, alopecia areata, male pattern baldness, Sweet's syndrome, Weber-Christian syndrome, erythema multiforme; infectious or noninfectious cellulitis; panniculitis; cutaneous lymphoma, nonmelanoma skin cancer or other dysplasia lesions; drug-induced disease including fixed drug eruption;

(3) Eye diseases, including blepharitis; conjunctivitis including perennial and vernal allergic conjunctivitis; iritis; anterior and posterior uveitis; choroiditis; retinal disease associated with autoimmune, denaturation or inflammation; ophthalmia including sympathetic ophthalmia; sarcoidosis; viral, fungal or bacterial infectious diseases;

(4) Genitourinary diseases, including nephritis including interstitial and glomerulonephritis; nephrotic syndrome; cystitis including acute or chronic (interstitial) cystitis and Hunner's ulcer; acute or chronic urethritis, prostatitis, epididymitis, oophoritis and salpingitis; vulvovaginitis; Peyronie's disease; erectile dysfunction (male and female);

(5) Allograft rejections, including acute and chronic rejection after transplantation of kidney, heart, liver, lung, bone marrow, skin or cornea, or after blood transfusion, etc.; or chronic graft-versus-host disease;

(6) Autoimmune diseases, including chronic rheumatoid arthritis, ulcerative colitis, systemic lupus erythematosus, multiple sclerosis, Hashimoto's thyroiditis, Grave's disease, Addison's disease, diabetes, idiopathic thrombocytopenic purpura, eosinophilic fasciitis, hyper IgE syndrome, or other autoimmune diseases and allergic diseases such as autoimmune disease syndrome including antiphospholipid antibody syndrome;

(7) Cancer diseases, including prostate cancer, breast cancer, lung cancer, uterus cancer, pancreas cancer, liver cancer, colon cancer, stomach cancer, skin cancer or brain tumor, and malignant bone marrow neoplasm (e.g., leukemia) and lymphoproliferative tumor such as Hodgkin's lymphoma or non-Hodgkin's lymphoma. It is useful for usual treatment of these cancer diseases and also for prevention or treatment of metastasis, tumor recurrence and paraneoplastic syndrome;

(8) Infectious diseases, including viral infectious diseases such as genital wart, common wart, plantar wart, hepatitis B, hepatitis C, herpes simplex viral disease, molluscum contagiosum, variola, acquired immune deficiency syndrome (HIV), or infectious diseases caused by human papillomavirus (HPV), cytomegalovirus (CMV), varicella-zoster virus (VZV), rhinovirus, adenovirus, coronavirus, influenza virus or parainfluenza virus; bacterial diseases such as tuberculosis, mycobacterium avium complex, or leprosy; other infectious diseases such as infectious diseases caused by various fungi, candida, chlamydia or aspergillus, cryptococcus meningitis, carinii pneumonia, cryptosporidiosis, histoplasmosis, toxoplasmosis, trypanosome infectious diseases, or leishmaniasis.

The adenine compound or a pharmaceutically acceptable salt thereof of the present invention is also useful as a vaccine adjuvant.

The adenine compound or a pharmaceutically acceptable salt thereof of the present invention has a TLR activating effect, more specifically a TLR7 activating effect. The adenine compound or a pharmaceutically acceptable salt thereof of the present invention also shows interferon-α- and interferon-γ-inducing activity, and IL-4/IL-5 producing inhibition activity, and acts as an agent with helper T cell type 1 (Th1 cell)/helper T cell type 2 (Th2 cell) selective immunoregulatory activity. In other words, it is preferably useful as a therapeutic or preventive agent for allergic diseases caused by Th2 cell such as asthma, COPD, allergic rhinitis, allergic conjunctivitis or atopic dermatitis due to its Th2 cell selective immunosuppressive action. On the other hand, owing to its immunostimulatory action, they are also useful as a therapeutic or preventive agent for various diseases, such as viral infectious diseases (e.g., cancer, hepatitis B, hepatitis C, acquired immune deficiency syndrome (HIV), human papillomavirus disease (HPV)), bacterial infectious diseases, skin diseases (e.g., psoriasis), etc.

The adenine compound or a pharmaceutically acceptable salt thereof of the present invention is useful for treatment of airway obstruction diseases/conditions such as asthma or COPD, or for reducing the risk of these diseases.

The adenine compound or a pharmaceutically acceptable salt thereof of the present invention may be orally or parenterally administered without any limitation to the dosage forms. For example, an oral preparation may include capsules, powders, tablets, granules, subtle granules, syrups, liquids, suspensions, etc., and a parenteral preparation may include injections, drips, eye-drops, preparations for intrarectal administration, inhalations, air sprays (e.g., liquid/suspensions for sprays, aerosols, dry powders or for cartridge sprays for inhalators or insufflators, etc.), lotions, gels, ointments, creams, transdermal absorbents, transmucosal absorbents, nasal preprations, eardrops, tapes, transdermal patches, cataplasms, external powders, etc. These preparations may be prepared according to a conventional technique, and may contain conventional carriers, excipients, binders, lubricants, stabilizers, disintegrants, buffers, solubilizing agents, isotonic agents, surfactants, antiseptic agents, perfumes, and further optionally contains two or more kinds of additives for preparations.

The adenine compound or a pharmaceutically acceptable salt thereof of the present invention may be incorporated with a pharmaceutically acceptable carrier in a manner known to those skilled in the art to prepare a pharmaceutical composition suitable for each dosage form. For example, the adenine compound or a pharmaceutically acceptable salt thereof may be formed into a pharmaceutical composition comprising as an active ingredient 0.05 to 99% by weight, preferably 0.05 to 80% by weight, more preferably 0.1 to 70% by weight, more preferably 0.1 to 50% by weight of the compound.

Among the oral preparations, liquid preparations such as emulsions and syrups may be prepared by optionally using additives for preparations including water; sugars such as sucrose, sorbit, fructose; ethanol; glycols such as polyethyleneglycol, propyleneglycol, glycerol; oils such as sesame oil, olive oil, soybean oil; preservative such as p-hydroxybenzoate; sweetener such as saccharin; thickener such as carboxymethylcellulose; flavors such as strawberry flavor, peppermint flavor, or colorants, etc.

Solid preparations such as capsules, tablets, powders, granules, etc. may be prepared by optionally compounding the following carriers. Specifically, they may be prepared by using excipient such as lactose, glucose, sucrose, sorbitol, mannitol (mannite), cellulose derivatives; disintegrant such as starch (e.g., potato starch, cornstarch, amylopectin), sodium alginate; lubricant such as magnesium stearate, calcium stearate, polyethyleneglycol, wax, paraffin, talc; binder such as polyvinyl alcohol, polyvinylpyrrolidone, hydroxypropylcellulose, gelatin; surfactant such as fatty acid ester; plasticizer such as glycerin, etc. Sugar coated tablets may be coated by concentrated carbohydrate solutions, optionally containing gum arabic, gelatin, talc, titanium oxide, etc., on tablet cores prepared by using the above carriers. Alternatively, tablets may be film-coated by appropriate polymers dissolved in organic solvents which may be easily distilled away.

Soft gelatin capsules may be prepared by, for example, compounding the present compound with vegetable oil or polyethyleneglycol. Hard gelatin capsules may be prepared by using granules of the present compound which may be prepared by optionally compounding any one of the above carriers.

Among the parenteral preparations, liquid preparations in the form of injections, drips, eye-drops, eardrops, etc. may be preferably prepared as sterile isotonic liquid preparations. For example, the injections may be prepared by using aqueous media comprising saline solution, glucose solution, or a mixture of saline solution and glucose solution. The preparations for intrarectal administration may be prepared by using carriers such as cacao butter, and usually prepared in the form of suppositories.

The ointments, creams and gels usually contain 0.01 to 10% by weight of the present compound, and to aqueous or oily base may be optionally added preferable thickener and/or gelatinizing agent and/or solvent. For example, the base includes water and/or oil such as liquid paraffin, vegetable oil such as peanut oil or castor oil, or solvent such as polyethyleneglycol. The thickener and gelatinizing agent include soft paraffin, aluminum stearate, cetostearyl alcohol, polyethyleneglycol, lanolin, bee wax, carboxypolymethylene and cellulose derivative and/or glyceryl monostearate and/or nonionic emulsifier.

The lotions usually contain 0.01 to 10% by weight of the present compound, and may be formulated by aqueous or oily base and may typically comprise emulsifier, stabilizer, dispersing agent, precipitation inhibitor or thickener.

The external powders usually contain 0.01 to 10% by weight of the present compound, and may be formed by preferable powder base such as talc, lactose or starch.

The drips may be formulated by aqueous or nonaqueous base and may contain dispersing agent, solubilizer, precipitation inhibitor or preservative.

The air spray (e.g., spray, aerosol, dry powder preparation, etc.) may be optionally formulated as aqueous solution or suspension, or aerosol delivered from pressurized pack such as quantitative dose inhalator by using, for example, a preferable liquefied propellant. Dry powder preparation may be also used.

The aerosol appropriate for inhalation may be either suspension or solution, and typically contains the present compound and any appropriate propellants such as fluorocarbon or hydrogen-containing chlorofluorocarbon or a mixture thereof. Specifically, it contains hydrofluoroalkane, particularly 1,1,1,2-tetrafluoroethane, heptafluoroalkane (HFA) such as 1,1,1,2,3,3,3-heptafluoro-n-propane, or a mixture thereof. The aerosol may optionally contain additional preparation excipient well-known to those skilled in the art such as surfactant (e.g., oleic acid or lecithin) and cosolvent (e.g., ethanol), etc. Specifically, it may include inhalator known as "Turbuhaler^{™}".

For example, capsule or cartridge of gelatin used in inhalator or insufflator may be formulated containing a powder mixture and preferable powder base such as lactose or starch for inhalation of the compound used in the present invention. Each capsule or cartridge usually contains 20 µg to 10 mg of the present compound. Alternatively, the compound used in the present invention may be provided without an excipient such as lactose.

In case of oral or nasal inhalation as pressurized HFA aerosol and dry powder preparation, etc., the adenine compound or a pharmaceutically acceptable salt thereof of the present invention may be finely ground into 10 µm or less to suspend in fatty acid with 8 to 20 carbon atoms or a salt thereof (e.g., oleic acid), bile acid salt, phospholipid, alkyl saccharide, fully-fluorinated or polyethoxylated surfactant, or other pharmaceutically acceptable dispersing agent.

It is preferable that the adenine compound in the present invention is parenterally administered as a preparation for local administration. Specifically, the preferable preparation includes ointment, lotion (solution or suspension), cream, gel, tape, transdermal patch, cataplasm, spray, aerosol, dry powder preparation, water/suspensions for cartridge sprays for inhalator or insufflator, eye-drops, eardrops, nasal drops, transdermal patches, lung absorbents, airway absorbents or external powders, etc.

In the preparation for local administration in the present invention, the ratio of the active compound used in the present invention is generally 0.001 to 10% by weight, preferably 0.005 to 1% by weight depending on the forms of preparations. The ratio used in powders for inhalation or ventilation is in the range of 0.1 to 5% by weight.

Each quantitative dose or "one-sprayed amount" in the aerosol preferably contains 20 µg to 2000 µg, preferably about 20 µg to 500 µg of the compound used in the present invention. The administration may be once or several times a day, for example 2, 3, 4 or 8 times a day, for example 1, 2 or 3 units each.

The pharmacological activity may be measured in any assessments well-known to those skilled in the art, preferably in vitro assessments. Specific measuring method includes the one discribed in Examples in the present specification.

The present invention also encompasses a combination therapy for treating diseases described in the present specification wherein the compound of the formula (1) or a pharmaceutically acceptable salt thereof, or a pharmaceutical composition comprising the compound of the formula (1) or a pharmaceutically acceptable salt thereof is sequentially or simultaneously administered in combination with 1 or more of other following medicaments.

Particularly, the medicaments for treating inflammatory disease, COPD, asthma and allergic rhinitis include TNF-α inhibitor such as anti TNF monoclonal antibody (e.g., Remicade, CDP-870 and adalimumab) or TNF receptor immunoglobulin molecule (e.g., enbrel); locally- or systemically-administered nonselective cyclooxygenase: COX-1/COX-2 inhibitor (e.g., piroxicam, diclofenac, propionic acids such as naproxen, flurbiprofen, fenoprofen, ketoprofen and ibuprofen, fenamate such as mefenamic acid, indomethacin, sulindac, azapropazone, pyrazolone such as phenylbutazone, salicylate salt such as aspirin), COX-2 inhibitor (e.g., meloxicam, celecoxib, rofecoxib, valdecoxib, lumarocoxib, parecoxib and etoricoxib); glucocorticoid which is administered locally, orally, intramuscularly, intravenously or intraarticularly; methotrexate, leflunomide; hydroxychloroquine, d-penicillamine, auranofin, or other parenteral or oral gold preparation, etc.

The present invention also encompasses a combination of the present compounds with leukotriene biosynthetic inhibitor, 5-lipoxygenase (5-LO) inhibitor or 5-lipoxygenase activated protein (FLAP) antagonist, for example zileutone; ABT-761; fenleutone; tepoxalin; Abbott-79175; Abbott-85761; N-(5-substituted)-thiophen-2-alkylsulfonamide; 2,6-di-tert-butylphenolhydrazone; methoxytetrahydropyrane such as Zeneca ZD-2138; SB-210661; pyridinyl-substituted-2-cyanonaphthalene compound such as L-739010; 2-cyanoquinoline compound such as L-746530; MK-591, MK-886 and BAY-X-1005, etc.

The present invention also encompasses a combination therapy of the present compound with leukotriene (LT) B4, LTC4, LTD4, LTE4 receptor antagonist selected from the following group:
phenothiazine compound such as L-651392; amidino compound such as CGS-25019c; benzoxalamine such as ontazolast; benzenecarboxyimidamide such as BIIL284/260; and compounds such as zafirlukast, ablukast, montelukast, pranlukast, Verlukast (MK-679), RG-12525, Ro-245913, iralukast (CGP45715A) and BAY-X-7195, etc.

The present invention also encompasses a combination therapy of the present compound with phosphodiesterase (PDE) inhibitor such as methylxanthanin including theophylline and aminophylline; selective PDE isoenzyme including PDE4 inhibitor, isoform PDE4D inhibitor or PDE5 inhibitor.

The present invention also encompasses a combination therapy of the present compound which is orally or topically administered with, for example, histamine H1 receptor antagonist such as cetirizine, loratadine, desloratadine, fexofenadine, acrivastine, terfenadine, astemizole, azelastine, levocabastine, chlorpheniramine, promethazine, cyclizine or mizolastine, etc.

The present invention also encompasses a combination therapy of the present compound with histamine type 2 receptor antagonists which protect gastrointestinal.

The present invention also encompasses a combination therapy of the present compound with histamine type 4 receptor antagonists.

The present invention also encompasses a combination therapy of the present compound with α1/α2 adrenaline receptor agonist and vasoconstrictive sympathetic stimulant such as propylhexedrine, phenylephrine, phenylpropanolamine, ephedrine, pseudoephedrine, naphazoline hydrochloride, oxymetazoline hydrochloride, tetrahydrozoline hydrochloride, xylometazoline hydrochloride, tramazoline hydrochloride, or ethyl norepinephrine hydrochloride.

The present invention also encompasses a combination therapy of the present compound with anticholinergic agent including muscarinic receptor (M1, M2 and M3) antagonist such as atropine, hyoscine, glycopyrrolate, ipratropium bromide; tiotropium bromide; oxytropium bromide; pirenzepine; or telenzepine.

The present invention also encompasses a combination therapy of the present compound with β-adrenaline receptor agonist including β receptor subtypes 1 to 4 such as isoprenaline, salbutamol, formoterol, salmeterol, terbutaline, orciprenaline, bitolterol mesylate or pirbuterol.

The present invention also encompasses a combination therapy of the present compound with chromone such as sodium cromoglycate or nedocromil sodium.

The present invention also encompasses a combination therapy of the present compound with insulin-like growth factor type 1 (IGF-1) mimic.

The present invention also encompasses a combination therapy of the present compound with inhaled glucocorticoid such as flunisolide, triamcinolone acetonide, beclomethasone dipropionate, budesonide, fluticasone propionate, ciclesonide or mometasone furoate.

The present invention also encompasses a combination therapy of the present compound with matrix metalloprotease inhibitor, specifically inhibitor of stromelysin, collagenase, gelatinase, aggrecanase, particularly collagenase-1 (MMP-1), collagenase-2 (MMP-8), collagenase-3 (MMP-13), stromelysin-1 (MMP-3), stromelysin-2 (MMP-10) and stromelysin-3 (MMP-11), MMP-9 or MMP-12.

The present invention also encompasses a combination therapy of the present compound with chemokine receptor regulators of antagonists of CCR1, CCR2, CCR2A, CCR2B, CCR3, CCR4, CCR5, CCR6, CCR7, CCR8, CCR9, CCR10 and CCR11 (CC family); CXCR1, CXCR2, CXCR3, CXCR4 and CXCR5 (C-X-C family); C-X3-C family such as CX3CR1.

The present invention also encompasses a combination therapy of the present compound with cytokine function regulator including cytokine or medicaments acting on cytokine signaling pathway, for example α-, β- and γ-interferon, interleukin (IL) including IL1 to 15, and interleukin antagonist or inhibitor.

The present invention also encompasses a combination therapy of the present compound with immunoglobulin (Ig), immunoglobulin preparations, or antibodies and antagonists regulating Ig functions such as anti IgE antibody (omalizumab).

The present invention also encompasses a combination therapy of the present compound with systemically- or locally-administered antiinflammatory drugs such as thalidomide or derivatives thereof, retinoid, dithranol or calcipotriol.

The present invention also encompasses a combination therapy of the present compound with antibacterial agents such as penicillin derivative, tetracycline, macrolide, β-lactam, fluoroquinolone, metronidazole and inhaled aminoglycoside; and antiviral agents including acyclovir, famciclovir, valaciclovir, ganciclovir, cidofovir, amantadine, rimantadine, ribavirin; zanamivir, oseltamavir; enzyme inhibitor such as indinavir, nelfinavir, ritonavir and saquinavir; nucleoside reverse transcriptase inhibitor such as didanosine, lamivudine, stavudine, zalcitabine, zidovudine; or nonnucleoside reverse transcriptase inhibitor such as nevirapine or efavirenz.

The present invention also encompasses a combination therapy of the present compound with medicaments known as therapeutic agents for cancer. Preferable agents include the following (i) to (ix).

(i) Antiproliferative agents/antitumor agents and a combination thereof used as a therapeutic agent for tumors, for example alkylating agents (e.g., cisplatin, carboplatin, cyclophosphamide, nitrogen mustard, melphalan, chlorambucil, busulfan, or nitrosourea); antimetabolite (e.g., fluoropyrimidine such as 5-fluorouracil and tegafur, antifolate such as raltitrexed, methotrexate, cytosine arabinoside, hydroxyurea, gemcitabine or paclitaxel); antineoplastic antibiotics (e.g., anthracycline such as adriamycin, bleomycin, doxorubicin, daunomycin, epirubicin, idarubicin, mitomycin-C, dactinomycin, or mithramycin); antimitotic agents (e.g., vinca alkaloid such as vincristine, vinblastine, vindesine or vinorelbine, taxoid such as taxol or taxotere); or topoisomerase inhibitors (e.g., epipodophyllotoxine such as etoposide, teniposide, amsacrine, topotecan or camptothecin).

(ii) Cytostatic agents including antiestrogens (e.g., tamoxifen, toremifene, raloxifene, droloxifene or iodoxifene, etc.), estrogen receptor down regulators (e.g., fulvestrant), antiandrogenic agents (e.g., bicalutamide, flutamide, nilutamide, or cyproterone acetate), LHRH antagonists or LHRH agonists (e.g., goserelin, leuprorelin or buserelin), progestogen (e.g., megestrol acetate), aromatase inhibitors (e.g., anastrozole, letrozole, vorazole or exemestane) and 5α-reductase inhibitors (e.g., finasteride).

(iii) Inhibiting agents of invasion of cancer cells (e.g., metalloprotease inhibitors such as marimastat or inhibitors of urokinase plasminogen activating receptor functions).

(iv) Growth factor function inhibitors, e.g., growth factor antibody, growth factor receptor antibody (e.g., anti-erbb2 antibody trastuzumab or anti-erbbl antibody cetuximab [C225]), farnesyl transferase inhibitors, tyrosine kinase inhibitors, or serine/threonine kinase function inhibitors; e.g., epidermal growth factor inhibitors (e.g., EGFR family tyrosine kinase inhibitors such as N-(3-chloro-4-fluorophenyl)-7-methoxy-6-(3-morpholinopropoxy)quinazolin-4-amine (Gefitinib, AZD 1839), N-(3-ethynylphenyl)-6,7-bis(2-methoxyethoxy)quinazolin-4-amine (erlotinib, OSI-774) or 6-acrylamide-N-(3-chloro-4-fluorophenyl)-7-(3-morpholinopropoxy)quinazolin-4-amine (CI1033)); e.g., platelet-derived growth factor family inhibitors; or hepatocellular growth factor family inhibitors.

(v) Antiangiogenic agents, for example inhibiting agents of activity of vascular endothelial cell growth factor (e.g., anti-vascular endothelial cell growth factor antibody bevacizumab, compounds disclosed in international publications: WO97/22596, WO97/30035, WO97/32856 or WO98/13354), or compounds acting in other mechanisms (e.g., linomid, integrin αvβ3 function inhibitors or angiostatin).

(vi) Vascular damaging agents such as combretastatin A4 or compounds disclosed in international publications: WO99/02166, WOOO/40529, WO00/41669, WO01/92224, WO02/04434 and WO02/08213.

(vii) Antisense therapeutics, for example antisense to the above targets such as ISIS2503, anti-ras antisense.

(viii) Gene therapy, for example aberrant gene exchanging approach such as aberrant p53 and aberrant BRCA1 or BRCA2, GDEPT (Gene-directed enzyme pro-drug therapy) approach using cytosine deaminase, thymidine kinase or bacterial nitroreductase enzyme, approach enhancing patients' tolerance for chemotherapy or radiotherapy such as multi drug resistance gene therapy.

(ix) Immunotherapy approach, for example approach for enhancing immunity to cancer cells of patients by exposuring cytokine such as interleukin 2, interleukin 4 or Granulocyte-Macrophage Colony Stimulating Factor (GM-CSF) ex-vivo or in-vivo, T cell anergy reducing approach, approach transplanting immune cells such as cytokine exposed dendritic cells, approach using cytokine exposed tumor cell line, and approach using anti-idiotypic antibody, etc.

The present invention is specifically described in the following Examples, but it is not limited thereto.

### EXAMPLES

The following compounds were prepared according to the preparations described in the present specification. The abbreviations in the present specification are as follows.
EtOAc: ethyl acetate
DCM: dichloromethane
NBS: N-bromosuccinimide
DMF: N,N-dimethylformamide
DMSO: dimethylsulfoxide
THF: tetrahydrofuran
TFA: trifluoroacetic acid
MS: mass spectrometry
APCI: Atmospheric Chemical Ionization Method
HCl: hydrochloric acid
In the reverse-phase HPLC, "Waters Symmetry C8, Xterra or Phenomenex Gemini columns" was used, and acetonitrile and buffer (e.g., aqueous ammonium acetate solution, aqueous ammonia solution, aqueous formic acid solution or aqueous trifluoroacetic acid solution) were used as an elution solvent. Column chromatography was carried out using silica gel.

### Example 1:

### Methyl N-[2-(6-amino-2-butoxy-7,8-dihydro-8-oxo-9H-purin-9-yl)-ethyl]glycinate

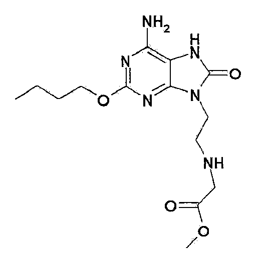

### (i) 2-Chloro-9-(tetrahydro-2H-pyran-2-yl)-9H-purin-6-amine

2,6-Dichloro-9-(tetrahydro-2H-pyran-2-yl)-9H-purine (55 g) was dissolved in 7N ammonia water/methanol (500 ml), and the mixture was heated in a sealed flask for 6 hours at 100°C. The reaction mixture was cooled to room temperature to let stand overnight. The titled compound was filtered to yield 40 g.
¹H NMR δ (CDCl₃) 8.02 (1H, s), 5.94 (2H, brs), 5.71 (1H, dd), 4.15-4.22 (1H, m), 3.75-3.82 (1H, m), 1.27-2.12 (6H, m).

### (ii) 2-Butoxy-9-(tetrahydro-2H-pyran-2-yl)-9H-purin-6-amine

The product (40 g) in step (i) was dissolved in 19% (w/w) sodium n-butoxide/butanol (250 ml). The reaction mixture was heated to reflux for 6 hours. The reaction suspension was cooled to room temperature, diluted with water and then extracted with diethyl ether. The combined organic layer was washed with water, dried and then concentrated under reduced pressure. The titled compound was crystallized in diethyl ether-isohexane and filtered to yield 19 g.
¹H NMR δ (CDCl₃) 7.87 (1H, s), 5.56-5.68 (3H, m), 4.31-4.35 (2H, t), 4.14-4.17 (1H, m), 3.76-3.80 (1H, m), 1.49-2.08 (10H, m), 0.98 (3H, t).

### (iii) 8-Bromo-2-butoxy-9-(tetrahydro-2H-pyran-2-yl)-9H-purin-6-amine

The product (30 g) in step (ii) was dissolved in dry dichloromethane (200 ml). The solution was stirred at room temperature, and thereto was added dropwise N-bromosuccinimide (NBS) (27 g). The reaction mixture was stirred overnight at room temperature, and then thereto was added 20% (w/w) sodium sulfate, and the separated aqueous layer was extracted with dichloromethane. The combined organic layer was washed with a saturated sodium bicarbonate and a saturated saline. The organic layer was concentrated under reduced pressure, and then the residue was dissolved in ethyl acetate. The mixture was washed with water, a saturated saline, and then dried. The solution was filtered through silica gel, and then concentrated under reduced pressure. The residue was triturated in diethyl ether and isohexane to filter the titled compound (26 g). The filtrate was concentrated under reduced pressure and the residue was purified by column chromatography (ethyl acetate/isohexane) to give a product (2.5 g). The resulting solid was collected to give a yellow solid. Yield: 28.5 g. Melting point: 148-150°C.
¹H NMR δ (CDCl₃) 5.59-5.64 (3H, m), 4.32 (2H, m), 4.17 (1H, m), 3.74 (1H, m), 3.08 (1H, m), 2.13 (1H, d), 1.48-1.83 (8H, m), 0.98 (3H, t).

### (iv) 2-Butoxy-8-methoxy-9-(tetrahydro-2H-pyran-2-yl)-9H-purin-6-amine

To anhydrous methanol (400 ml) was added sodium (3.7 g) under nitrogen flow. Thereto was added the product (28.5 g) in step (iii), and the reaction mixture was stirred at 65°C for 9 hours. The reaction mixture was concentrated under reduced pressure, and thereto was added water. The aqueous layer was extracted with ethyl acetate, and washed with a saturated saline and then dried. The mixture was crystallized by diethyl ether to give the titled compound. Yield: 14.2 g.
¹H NMR δ (CDCl₃) 5.51 (1H, dd), 5.28 (2H, brs), 4.29 (2H, t), 4.11-4.14 (4H, m), 3.70 (1H, m), 2.76-2.80 (1H, m), 2.05 (1H, d), 1.47-1.81 (8H, m), 0.97 (3H, t).

### (v) 2-Butoxy-8-methoxy-9H-purin-6-amine, TFA salt

The product (24 g) in step (iv) was dissolved in anhydrous methanol (300 ml), and thereto was added TFA (30 ml). The reaction mixture was stirred at room temperature for 3 days, and then concentrated under reduced pressure. The mixture was triturated in methanol/ethyl acetate to give the titled compound as a white crystal. Yield: 21 g.
¹H NMR δ (CD₃OD) 4.48 (2H, t), 4.15 (3H, s), 1.80 (2H, quintet), 1.50 (2H, sextet), 0.99 (3H, t).

### (vi) 9-(2-Bromoethyl)-2-butoxy-8-methoxy-9H-purin-6-amine

To a mixture of potassium carbonate (3.7 g) and 1,2-dibromoethane (0.6 ml) in DMF (20 ml) was added dropwise the product (2 g) in step (v) over 10 minutes at room temperature rapidly stirring, and the mixture was stirred for 1.5 hours. The reaction mixture was diluted with water and extracted with ethyl acetate. The combined extract was washed with a saturated saline and dried. The reaction mixture was purified by column chromatography to give the titled compound as a white solid. Yield: 1.2 g.
¹H NMR δ (CDCl₃) 5.15 (2H, s), 4.30 (4H, m), 4.13 (3H, s), 3.65 (2H, t), 1.82-1.72 (2H, m), 1.56-1.43 (2H, m), 0.97 (3H, t).

### (vii) tert-Butyl N-[2-(6-amino-2-butoxy-8-methoxy-9H-purin-9-yl)ethyl]glycinate

The product (0.3. g) in step (vi) and glycine t-butyl ester (0.5 g) were suspended in dry DMF (1 ml) and acetonitrile (5 ml), and heated at 70°C for 48 hours. The reaction mixture was cooled to room temperature and diluted with a saturated saline (50 ml), and then extracted with ethyl acetate. The collected extract was dried and concentrated under reduced pressure, and the residue was purified by column chromatography to give the titled compound. Yield: 0.27 g.
¹H NMR δ (CDCl₃) 5.18 (2H, s), 4.28 (2H, t), 4.12 (3H, s), 4.05 (2H, t), 3.31 (2H, s), 2.99 (2H, t), 1.81-1.69 (2H, m), 1.55-1.44 (2H, m), 1.44 (9H, s), 0.96 (3H, t).

### (viii) Methyl N-[2-(6-amino-2-butoxy-7,8-dihydro-8-oxo-9H-purin-9-yl)ethyl]glycinate

To a solution of the product (0.25 g) obtained in step (vii) in methanol (10 ml) was added trimethylsilyl chloride (5 ml), and the mixture was stirred for 24 hours under refluxing. The reaction mixture was cooled to room temperature to let stand for 1 day, and a colorless solid was filtered to give the titled compound. Yield: 0.23 g.
¹H NMR δ (DMSO) 10.92 (1H, s), 9.36 (2H, s), 4.24 (2H, t), 4.10-4.02 (4H, m), 3.75 (3H, s), 3.30 (2H, s), 1.67 (2H, quintet), 1.41 (2H, sextet), 0.93 (3H, t).
MS: APCI (+ve): 339 (M+H)

### Example 2:

### Methyl (2S)-2-{[2-(6-amino-2-butoxy-8-oxo-7,8-dihydro-9H-purin-9-yl)ethyl]amino}butanoate

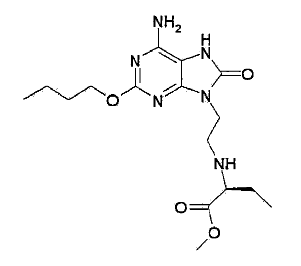

The titled compound was prepared by using tert-butyl (2S)-2-butanoate in the similar manner to Example 1. The titled compound was recrystallized by acetonitrile/methanol to give a colorless solid. Yield: 0.24 g.
¹H NMR δ (DMSO) 9.82 (1H, s), 6.38 (2H, s), 4.14 (2H, t), 3.81-3.62 (2H, m), 3.59 (3H, s), 3.20 (1H, t), 2.87-2.75 (1H, m), 2.71-2.59 (1H, m), 2.25-2.04 (1H, m), 1.64 (2H, quintet), 1.56-1.31 (4H, m), 0.92 (3H, t), 0.77 (3H, t).
MS: APCI (+ve): 367 (M+H)

### Example 3:

### Methyl N-[2-(6-amino-2-butoxy-8-oxo-7,8-dihydro-9H-purin-9-yl)ethyl]-L-alaninate

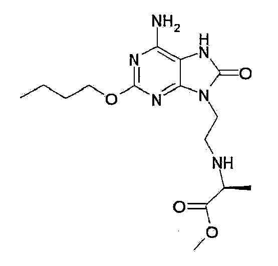

### (i) tert-Butyl N-[2-(6-amino-2-butoxy-8-methoxy-9H-purin-9-yl) ethyl]-L-alaninate

The product (0.30 g) in step (vi) in Example 1, L-alanine tert-butyl ester hydrochloride (0.32 g) and potassium carbonate (0.245 g) were suspended in DMF (2 ml), and heated at 65°C for 20 hours. The reaction mixture was cooled to room temperature, and concentrated under reduced pressure. Yield: 0.10 g.

### (ii) Methyl N-[2-(6-amino-2-butoxy-8-oxo-7,8-dihydro-9H-purin-9-yl) ethyl]-L-alaninate

The product in step (i) was treated in the similar manner to step (viii) in Example 1 to prepare the titled compound. The mixture was purified by reverse-phase HPLC to give the titled compound as a colorless solid. Yield: 0.075 g.
¹H NMR DMSO-d₆: δ 9.82 (1H, s), 6.38 (2H, s), 4.14 (2H, t), 3.79-3.62 (2H, m), 3.59 (3H, s), 3.42-3.32 (1H, m), 2.85-2.75 (1H, m), 2.73-2.63 (1H, m), 2.26-2.12 (1H, m), 1.64 (2H, quintet), 1.39 (2H, sextet), 1.12 (3H, d), 0.92 (3H, t).

### Example 4:

### Methyl N-[2-(6-amino-2-butoxy-8-oxo-7,8-dihydro-9H-purin-9-yl)ethyl]-2-methylalaninate

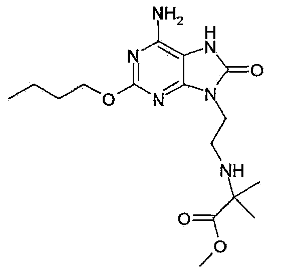

The titled compound was prepared by using tert-butyl 2-methyl alaninate in the similar manner to Example 1. The mixture was purified by reverse-phase HPLC to give the titled compound as a colorless solid. Yield: 0.13 g.
¹H NMR δ (CDCl₃) 4.28 (2H, t), 3.94 (2H, t), 3.56 (3H, s), 2.81 (2H, t), 1.73 (2H, quintet), 1.48 (2H, sextet), 1.25 (6H, s), 0.98 (3H, t).
MS: APCI (+ve): 367 (M+H)

### Example 5:

### Methyl N-[2-(6-amino-2-butoxy-8-oxo-7,8-dihydro-9H-purin-9-yl)ethyl]-D-valinate

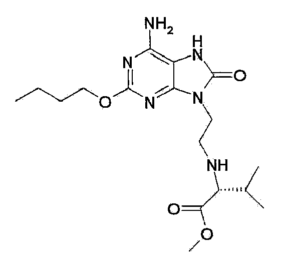

The titled compound was prepared by using D-valine tert-butyl ester in the similar manner to Example 1. The residue was purified by RP HPLC to give the titled compound as a colorless solid. Yield: 0.070 g. ¹H NMR δ (DMSO) 9.82 (1H, s), 6.37 (2H, s), 4.15 (2H, t), 3.80-3.63 (2H, m), 3.59 (3H, s), 3.03-2.94 (1H, m), 2.88-2.78 (1H, m), 2.66-2.56 (1H, m), 2.12-2.03 (1H, m), 1.74 (1H, quintet), 1.68-1.60 (2H, m), 1.39 (2H, sextet), 0.92 (3H, t), 0.79 (6H, dd).
MS: APCI (+ve): 367 (M+H)

### Example 6:

### Dimethyl N-[2-(6-amino-2-butoxy-8-oxo-7,8-dihydro-9H-purin-9-yl) ethyl] -L-aspartate

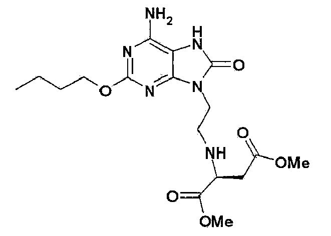

### (i) tert-Butyl N²-[2-(6-amino-2-butoxy-8-oxo-7,8-dihydro-9H-purin-9-yl)ethyl]-L-asparatate

The product (0.30 g) in step (vi) in Example 1 and L-aspartic acid tert-butyl ester (0.66 g) were heated in acetonitrile (3 ml) at 70°C for 6 days. The reaction mixture was concentrated under reduced pressure. The residue was purified by RP HPLC to give the titled compound as a viscous solid. Yield: 0.28 g.
¹H NMR δ (DMSO) 7.33 (1H, s), 7.06 (1H, s), 6.74 (2H, s), 4.16 (2H, t), 4.03 (3H, s), 3.87 (2H, t), 2.85-2.69 (2H, m), 2.46 (1H, dd), 2.23 (1H, dd), 2.19-2.11 (1H, m), 1.64 (2H, quintet), 1.40 (2H, sextet), 1.33 (9H, s), 0.92 (3H, t).

### (ii) Dimethyl N-[2-(6-amino-2-butoxy-8-oxo-7,8-dihydro-9H-purin-9-yl)ethyl]-L-aspartate

The titled compound was prepared from the product in step (i) in the similar manner to step (viii) in Example 1. The product was purified by reverse-phase HPLC. Yield: 0.14 g.
¹H NMR δ (CDCl₃) 10.71 (1H, s), 6.01 (2H, s), 4.27 (2H, t), 4.00-3.91 (2H, m), 3.75-3.66 (4H, m), 3.63 (3H, s), 3.17-3.06 (1H, m), 2.99-2.88 (1H, m), 2.76-2.60 (2H, m), 2.26 (1H, s), 1.75 (2H, quintet), 1.48 (2H, sextet), 0.96 (3H, t).
MS: APCI (+ve): 411 (M+H)

### Example 7:

### Methyl N²-[2-(6-amino-2-butoxy-8-oxo-7,8-dihydro-9H-purin-9-yl) ethyl] -L-lysinate

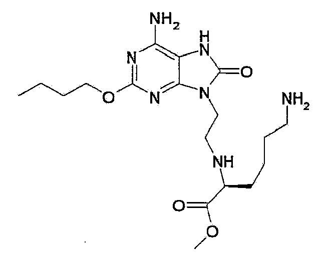

The titled compound was prepared by using N⁶-[(benzyloxy)-carbonyl]-L-lysine tert-butyl ester in the similar manner to Example 6. The residue was purified by reverse-phase HPLC. Yield: 0.050 g.
¹H NMR δ (CDCl₃) 4.30 (2H, t), 3.94 (2H, t), 3.68 (3H, s), 3.36-3.32 (1H, m), 3.05-2.93 (1H, m), 2.91-2.79 (1H, m), 2.66 (2H, t), 1.82-1.70 (2H, m), 1.69-1.41 (6H, m), 1.40-1.24 (2H, m), 1.01 (3H, t).
MS: APCI (+ve): 410 (M+H)

### Example 8:

### Methyl (2S)-{[2-(6-amino-2-butoxy-8-oxo-7,8-dihydro-9H-purin-9-yl) ethyl] amino}(4-hydroxyphenyl) acetate

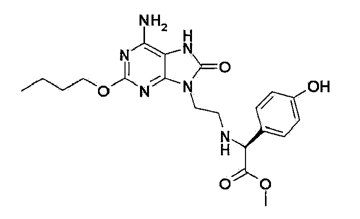

The product (0.3 g) in step (vi) in Example 1, (2S)-amino-(4-hydroxyphenyl)-acetic acid (0.34 g) and potassium carbonate (0.56 g) were heated in DMSO (5 ml) at 70°C for 4 hours. The reaction mixture was cooled to room temperature and dissolved in methanol (200 ml), and thereto was added trimethylsilyl chloride (40 ml). The reaction mixture was heated for 17 hours under refluxing. The reaction mixture was concentrated under reduced pressure. The residue was purified by reverse-phase HPLC to give the titled compound as a creamy solid. Yield: 0.045 g.
¹H NMR δ (DMSO) 9.82 (1H, s), 7.09 (2H, d), 6.67 (2H, d), 6.37 (2H, s), 4.36 (1H, s), 4.12 (2H, t), 3.74 (2H, t), 3.54 (3H, s), 2.70 (2H, t), 1.63 (2H, quintet), 1.39 (2H, sextet), 0.92 (3H, t).
MS: APCI (+ve): 431 (M+H).

### Example 9:

### Methyl N-[2-(6-amino-2-butoxy-8-oxo-7,8-dihydro-9H-purin-9-yl)ethyl]-N-methyl glycinate

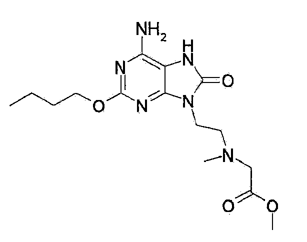

A mixture of the product (0.3 g) in step (vi) in Example 1, sarcosine tert-butyl ester hydrochloride (635 mg) and N,N-diisopropylethylamine (0.61 ml) was heated in DMSO (2 ml) at 55°C for 7 hours. To the reaction mixture was added methanol (10 ml), and then thereto was added 4M HCl/dioxane (10 ml). The mixture was heated at 55°C overnight, and then the reaction mixture was partitioned by water/ethyl acetate. The organic layer was separated and dried, and then concentrated under reduced pressure. The residue was purified by silica gel column chromatography, and eluted with ethyl acetate. Yield: 0.11 g.
¹H NMR DMSO-d₆: δ 9.85 (1H, s); 6.39 (2H, s); 4.14 (2H, t); 3.75 (2H, t); 3.55 (3H, s); 3.32 (2H, s); 2.81 (2H, t); 2.23 (3H, s); 1.64 (2H, quintet); 1.38 (2H, sextet); 0.92 (3H, t).
MS: APCI (+ve): 353 (M+H)

### Example 10:

### Methyl N-[3-(6-amino-2-butoxy-8-oxo-7,8-dihydro-9H-purin-9-yl)propyl]-O-[3-(dimethylamino)propyl]-L-tyrosinate

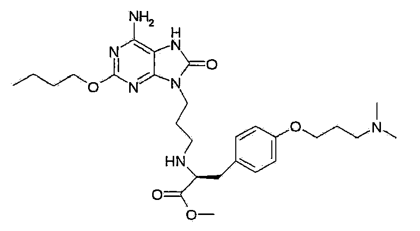

### (i) tert-Butyl O-[3-(dimethylamino)propyl]-L-tyrosinate

To a solution of triphenylphosphine (2.65 g) and L-tyrosine tert-butyl ester (2 g) in anhydrous THF (50 ml) was added dropwise diisopropyl (E)-diazene-1,2-dicarboxylate (1.7 ml) at 5°C. 10 minutes after the addition, thereto was added dimethylaminopropanol (1.2 ml), and the reaction mixture was stirred at room temperature for 17 hours. The solvent was distilled away under reduced pressure, and the residue was purified by silica gel column chromatography, and eluted with 5% 3N ammonia water/MeOH-dichloromethane. Yield: 1.69 g.
¹H NMR δ (CDCl₃) 7.11 (2H, d), 6.83 (2H, d), 3.99 (2H, t), 3.55 (1H, t), 2.97 (1H, dd), 2.77 (1H, dd), 2.44 (2H, t), 1.94 (2H, quintet), 1.44 (9H, s).

### (ii) 9-(3-Bromopropyl)-2-butoxy-8-methoxy-9H-purin-6-amine

The titled compound was prepared by using 1,3-dibromopropane in the similar manner to step (vi) in Example 1. Yield: 16 g.
¹H NMR δ (CDCl₃) 5.19 (2H, s), 4.28 (2H, J = 6.7 Hz, t), 4.12 (3H, s), 4.09 (2H, J = 9.4 Hz, t), 3.37 (2H, J = 13.3Hz, t), 2.39-2.30 (2H, m), 1.81-1.72 (2H, m), 1.55-1.43 (2H, m), 0.96 (3H, J = 11.4 Hz, t).

### (iii) Methyl N-[3-(6-amino-2-butoxy-8-oxo-7,8-dihydro-9H-purin-9-yl)propyl]-O-[3-(dimethylamino)propyl]-L-tyrosinate

The titled compound was prepared by using the products in steps (i) and (ii) in the similar manner to steps (vii) and (viii) in Example 1. Yield: 0.04 g.
¹H NMR δ (CD₃OD) 7.03 (2H, d), 6.80 (2H, d), 4.27 (2H, t), 3.97 (2H, t), 3.84 (2H, t), 3.55 (3H, s), 3.44 (1H, dd), 2.91-2.76 (2H, m), 2.64-2.42 (4H, m), 2.28 (6H, s), 1.98-1.83 (4H, m), 1.73 (2H, quintet), 1.48 (2H, sextet), 0.97 (3H, t).
MS: APCI (+ve): 544 (M+H)

### Example 11:

### Methyl N-[4-(6-amino-2-butoxy-8-oxo-7,8-dihydro-9H-purin-9-yl)butyl]-L-alaninate

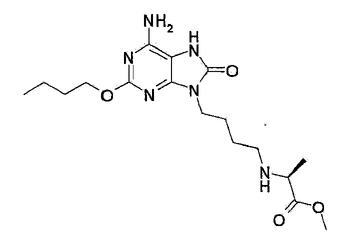

### (i) 9-(4-Bromobutyl)-2-butoxy-8-methoxy-9H-purin-6-amine

The titled compound was prepared by using 1,4-dibromobutane in the similar manner to step (vi) in Example 1.
¹H NMR δ (DMSO-d₆) 6.77 (2H, s), 4.17 (2H, t), 4.05 (3H, s), 3.86 (2H, t), 3.55 (2H, t), 1.85-1.69 (6H, m), 1.68-1.60 (2H, m), 1.44-1.34 (2H, m), 0.91 (3H, t).

### (ii) Methyl N-[4-(6-amino-2-butoxy-8-oxo-7,8-dihydro-9H-purin-9-yl)butyl]-L-alaninate

The titled compound was prepared by using the product in step (i) and L-alanine tert-butyl ester in the similar manner to steps (vii) and (viii) in Example 1. Yield: 6 mg.
¹H NMR DMSO-d₆: δ 9.93 (1H, s), 6.59 (1H, s), 6.42 (2H, s), 4.18-4.11 (2H, m), 3.69-3.61 (5H, m), 3.37-3.28 (1H, m), 2.49-2.40 (2H, m), 1.72-1.58 (4H, m), 1.44-1.31 (4H, m), 1.17 (3H, d), 0.92 (3H, t).
MS: APCI (+ve): 381 (M+H)

### Example 12:

### Methyl N-[4-(6-amino-2-butoxy-8-oxo-7,8-dihydro-9H-purin-9-yl)-butyl]-N-methyl glycinate

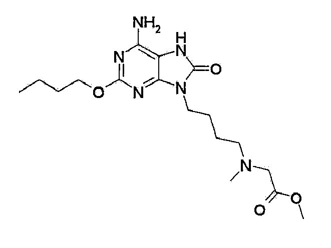

The titled compound was prepared by using the product in step (i) in Example 11 and N-methylsarcosine methyl ester in the similar manner to Example 3. Yield: 39 mg.
¹H NMR DMSO-d₆: δ 9.84 (1H, s), 6.38 (2H, s), 4.14 (2H, t), 3.66 (2H, t), 3.59 (3H, s), 3.21 (2H, s), 2.43 (2H, t), 2.21 (3H, s), 1.64 (4H, quintet), 1.43-1.32 (4H, m), 0.92 (3H, t).
MS: APCI (+ve): 381 (M+H)

### Example 13:

### N-[2-(6-Amino-2-butoxy-8-oxo-7,8-dihydro-9H-purin-9-yl)ethyl]-L-alanine

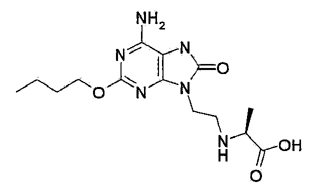

¹H NMR δ (DMSO + DCl) 4.36 (2H, t), 4.14-4.05 (2H, m), 3.40-3.25 (2H, m), 1.70 (2H, quintet), 1.48-1.36 (5H, m), 0.94 (3H, t)

### Example 14:

### N-[2-(6-Amino-2-butoxy-8-oxo-7,8-dihydro-9H-purin-9-yl)ethyl]-D-valine

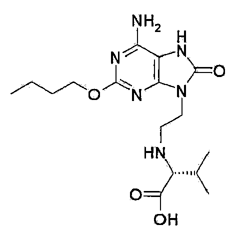

¹H NMR DMSO-d₆: δ 9.99 (1H, s), 6.45 (2H, s), 4.15 (2H, t), 3.88-3.73 (2H, m), 3.05-2.92 (2H, m), 2.83-2.73 (1H, m), 1.91-1.82 (1H, m), 1.63 (2H, quintet), 1.39 (2H, sextet), 0.92 (3H, t), 0.85 (6H, d)

### Example 15:

### N-[2-(6-Amino-2-butoxy-8-oxo-7,8-dihydro-9H-purin-9-yl)ethyl]glycine

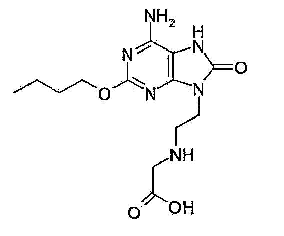

¹H NMR δ (D₂O + DCl) 4.34 (2H, t), 4.15-4.06 (2H, m), 3.85 (2H, s), 3.40-3.32 (2H, m), 1.57 (2H, quintet), 1.22 (2H, sextet), 0.71 (3H, t)

### Example 16:

### (2S)-2-{[2-(6-Amino-2-butoxy-8-oxo-7,8-dihydro-9H-purin-9-yl) ethyl] amino}butanoic acid

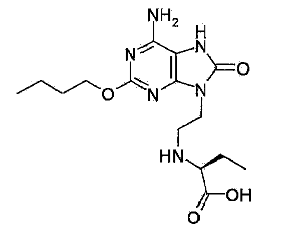

¹H NMR δ (CDCl₃) 5.14 (2H, s), 4.28 (2H, t), 4.11 (3H, s), 4.03 (2H, t), 2.85 (2H, t), 1.79-1.68 (2H, m), 1.49 (2H, sextet), 1.33 (9H, s), 1.21 (6H, s), 0.96 (3H, t).

### Example 17:

### N-[2-(6-Amino-2-butoxy-8-oxo-7,8-dihydro-9H-purin-9-yl)ethyl]-2-methylalanine

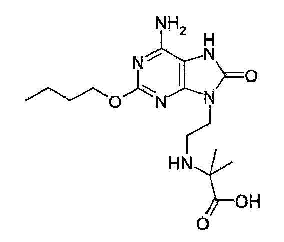

¹H NMR DMSO-d₆: δ 6.46 (2H, s), 4.11 (2H, t), 3.72-3.65 (2H, m), 2.66-2.59 (2H, m), 1.62 (2H, quintet), 1.39 (2H, sextet), 1.02 (6H, s), 0.92 (3H, t)

### Example 18:

### N-[2-(6-Amino-2-butoxy-8-oxo-7,8-dihydro-9H-purin-9-yl)ethyl]-N-methylglycine

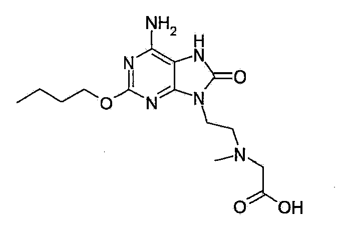

¹H NMR DMSO-d₆: δ 9.87 (1H, s); 6.39 (2H, s); 4.14 (2H, t); 3.75 (2H, t); 3.55 (3H, s); 3.32 (2H, s); 2.81 (2H, t); 2.23 (3H, s); 1.64 (2H, quintet); 1.38 (2H, sextet); 0.92 (3H, t)

### Example 19: Human TLR7 reporter assay

Human TLR7 or rat TLR7 plasmid and reporter plasmid (NF-kB-SEAP) stably-induced HEK293 cells were suspended in DMEM media (10% FBS, 1% NEAA, 10 ug/mL blastocidin S HCl, 100 ug/mL Zeocin), and the suspension was seeded in 90 µl/well on 96 well plate (hTLR7/seap-293: 20000 cells/well, rTLR7/seap-293: 25000 cells/well).
To the cells seeded on 96 well plate was added in 10 µl/well a test compound wherein DMSO stock solution (2 µl) was hundredfold diluted with medium (200 µl) (final concentration; 1 nM-10 µM, common ratio 3). The mixture was stirred tapping in the side of the plate, and then incubated for 20 hours in CO2 incubator. To the cells stimulated by a test compound was added a substrate for reporter assay (substrate for SEAP, pNPP) by 50 µl/well. 10 minutes after the addition of substrate, a quenching solution (4N NaOH) was added by 50 µl/well, and the enzymatic reaction was quenched. Top seal A was attached on the plate, and absorbance was measured by microplate reader (405 nm).

Each human TLR7 binding activity (EC50) of each compound is shown in Table 1.

**Table 1**

| Compound | EC50 (nM) |
|---|---|
| Example 1 | 289.9 |
| Example 6 | 880.8 |
| Example 7 | 1710.5 |

## Claims

1. An adenine compound of the formula (1): 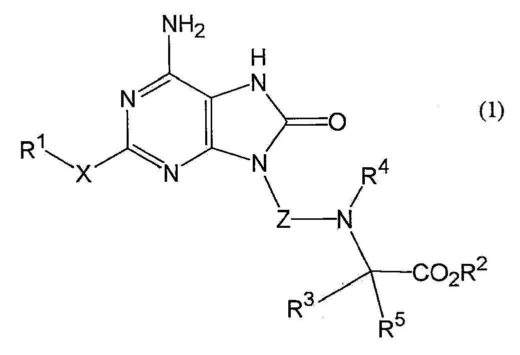
wherein Z is substituted or unsubstituted alkylene, or a single bond, in which any 1 to 3 of methylene group(s) in the alkylene may be replaced by oxygen, sulfur, SO, SO₂ or carbonyl groups;
R¹ is substituted or unsubstituted alkyl, substituted or unsubstituted alkenyl, substituted or unsubstituted alkynyl, substituted or unsubstituted cycloalkyl, substituted or unsubstituted aryl, or substituted or unsubstituted heteroaryl group;
R² is hydrogen, or substituted or unsubstituted alkyl group;
R³, R⁴ and R⁵ are independently hydrogen, substituted or unsubstituted alkyl, substituted or unsubstituted alkenyl, substituted or unsubstituted alkynyl, substituted or unsubstituted aryl, or substituted or unsubstituted heteroaryl, or R³ and R⁵ may be combined together with the adjacent carbon atom to form substituted or unsubstituted 3- to 7-membered saturated carbocycle or heterocycle;
X is oxygen, sulfur, SO₂, NR⁶, wherein R⁶ is hydrogen or alkyl group, or a single bond; or a pharmaceutically acceptable salt thereof.

2. The adenine compound of claim 1, wherein X is oxygen, sulfur, SO₂, NR⁶, wherein R⁶ is hydrogen or alkyl group with 1 to 6 carbon atom(s), or a single bond;
Z is alkylene with 2 to 6 carbon atoms wherein the alkylene may be substituted with halogen, hydroxyl, alkoxy with 1 to 6 carbon atom(s), 6- to 10-membered aryl or 5- to 10-membered heteroaryl, wherein the aryl group and the heteroaryl group may be substituted with 1 or more substituent(s) independently selected from halogen, hydroxyl, alkyl with 1 to 6 carbon atom(s), alkoxy with 1 to 6 carbon atom(s), haloalkyl with 1 to 6 carbon atom(s), haloalkoxy with 1 to 6 carbon atom(s), and amino optionally substituted with the same or different 1 or 2 alkyl(s) with 1 to 6 carbon atom(s);
R¹ is substituted or unsubstituted alkyl with 1 to 6 carbon atom(s), substituted or unsubstituted alkenyl with 2 to 6 carbon atoms, substituted or unsubstituted alkynyl with 2 to 6 carbon atoms, substituted or unsubstituted 6- to 10-membered aryl, substituted or unsubstituted 5- to 10-membered heteroaryl, or substituted or unsubstituted 3- to 8-membered cycloalkyl;
R2 is hydrogen, or substituted or unsubstituted alkyl with 1 to 6 carbon atom(s);
R³, R⁴ and R⁵ are independently hydrogen, substituted or unsubstituted alkyl with 1 to 6 carbon atom(s), substituted or unsubstituted alkenyl with 2 to 6 carbon atoms, substituted or unsubstituted alkynyl with 2 to 6 carbon atoms, substituted or unsubstituted 6- to 10-membered aryl, or substituted or unsubstituted 5- to 10-membered heteroaryl, or R³ and R⁵ may be combined together with the adjacent carbon atom to form substituted or unsubstituted 3-to 7-membered saturated carbocycle, or substituted or unsubstituted 4-to 7-membered saturated nitrogen-containing heterocycle;
the substituted alkyl, substituted alkenyl and substituted alkynyl are substituted with 1 or more substituent(s) independently selected from the following (a) to (c):
(a) halogen, hydroxyl, carboxy, haloalkyl with 1 to 6 carbon atom(s), haloalkoxy with 1 to 6 carbon atom(s), mercapto;
(b) alkoxy with 1 to 6 carbon atom(s), alkylthio with 1 to 6 carbon atom(s), alkylcarbonyl with 2 to 6 carbon atoms, alkylcarbonyloxy with 2 to 6 carbon atoms, alkoxycarbonyl with 2 to 6 carbon atoms, alkylsulfonyl with 1 to 6 carbon atom(s), alkylsulfinyl with 1 to 6 carbon atom(s), alkenyloxy with 2 to 6 carbon atoms, alkenylthio with 2 to 6 carbon atoms, alkenylcarbonyl with 3 to 6 carbon atoms, alkenylcarbonyloxy with 3 to 6 carbon atoms, alkenyloxycarbonyl with 3 to 6 carbon atoms, alkenylsulfonyl with 2 to 6 carbon atoms, alkenylsulfinyl with 2 to 6 carbon atoms, alkynyloxy with 2 to 6 carbon atoms, alkynylthio with 2 to 6 carbon atoms, alkynylcarbonyl with 3 to 6 carbon atoms, alkynylcarbonyloxy with 3 to 6 carbon atoms, alkynyloxycarbonyl with 3 to 6 carbon atoms, alkynylsulfonyl with 2 to 6 carbon atoms, alkynylsulfinyl with 2 to 6 carbon atoms,
wherein each group may further be substituted by 1 or more substituent(s) independently selected from halogen, hydroxyl, alkoxy with 1 to 6 carbon atom(s), carboxy, alkoxycarbonyl with 2 to 6 carbon atoms, alkylsulfonyl with 1 to 6 carbon atom(s), amino optionally substituted by the same or different 1 or 2 alkyl(s) with 1 to 6 carbon atom(s), carbamoyl optionally substituted by the same or different 1 or 2 alkyl(s) with 1 to 6 carbon atom(s), and sulfamoyl optionally substituted by the same or different 1 or 2 alkyl(s) with 1 to 6 carbon atom(s);
(c) amino, carbamoyl or sulfamoyl which may be independently substituted by 1 or 2 substituent(s) described in the following (j) to (l), 6- to 10-membered aryl, 6- to 10-membered aryloxy, 6- to 10-membered arylcarbonyl, 6- to 10-membered arylcarbonyloxy, 6- to 10-membered aryloxycarbonyl, 6- to 10-membered arylsulfonyl, 6- to 10-membered arylsulfinyl, 5- to 10-membered heteroaryl, 5- to 10-membered heteroaryloxy, 5- to 10-membered heteroarylcarbonyl, 5- to 10-membered heteroarylcarbonyloxy, 5- to 10-membered heteroaryloxycarbonyl, 5- to 10-membered heteroarylsulfonyl or 5- to 10-membered heteroarylsulfinyl which may be independently substituted by 1 or more substituent(s) described in the following (g) to (i), or 3- to 8-membered cycloalkyl, 3- to 8-membered cycloalkoxy, 3- to 8-membered cycloalkylcarbonyl, 3- to 8-membered cycloalkylcarbonyloxy, 3- to 8-membered cycloalkoxycarbonyl, 3- to 8-membered cycloalkylsulfonyl, 3- to 8-membered cycloalkylsulfinyl or 4-to 8-membered saturated heterocycle which may be independently substituted by 1 or more substituent(s) described in the following (d) to (f);
the substituted cycloalkyl and the substituted 3- to 8-membered saturated carbocycle and substituted 4- to 8-membered saturated nitrogen-containing heterocycle which both of R³ and R⁵ are combined to form are substituted by 1 or more substituent(s) independently selected from the following (d) to (f):
(d) halogen, hydroxyl, carboxy, mercapto, haloalkyl with 1 to 6 carbon atom(s), haloalkoxy with 1 to 6 carbon atom(s);
(e) alkyl with 1 to 6 carbon atom(s), alkenyl with 2 to 6 carbon atoms, alkynyl with 2 to 6 carbon atoms, alkoxy with 1 to 6 carbon atom(s), alkylthio with 1 to 6 carbon atom(s), alkylcarbonyl with 2 to 6 carbon atoms, alkylcarbonyloxy with 2 to 6 carbon atoms, alkoxycarbonyl with 2 to 6 carbon atoms, alkylsulfonyl with 1 to 6 carbon atom(s), alkylsulfinyl with 1 to 6 carbon atom(s),
wherein each group may further be substituted by 1 or more substituent(s) independently selected from halogen, hydroxyl, alkoxy with 1 to 6 carbon atom(s), carboxy, alkoxycarbonyl with 2 to 6 carbon atoms, alkylsulfonyl with 1 to 6 carbon atom(s), amino optionally substituted by the same or different 1 or 2 alkyl(s) with 1 to 6 carbon atom(s), carbamoyl optionally substituted by the same or different 1 or 2 alkyl(s) with 1 to 6 carbon atom(s), and sulfamoyl optionally substituted by the same or different 1 or 2 alkyl(s) with 1 to 6 carbon atom(s);
(f) 6- to 10-membered aryl or 5- to 10-membered heteroaryl which may be independently substituted by 1 or more substituent(s) described in the following (g) to (i), or amino, carbamoyl or sulfamoyl which may be independently substituted by 1 or 2 substituent(s) described in the following (j) to (l);
the substituted aryl and the substituted heteroaryl are substituted by 1 or more substituent(s) independently selected from the following (g) to (i):
(g) halogen, hydroxyl, mercapto, cyano, nitro, haloalkyl with 1 to 6 carbon atom(s), haloalkoxy with 1 to 6 carbon atom(s);
(h) alkyl with 1 to 6 carbon atom(s), alkoxy with 1 to 6 carbon atom(s), alkylthio with 1 to 6 carbon atom(s), alkylcarbonyl with 2 to 6 carbon atoms, alkylcarbonyloxy with 2 to 6 carbon atoms, alkylsulfonyl with 1 to 6 carbon atom(s), alkylsulfinyl with 1 to 6 carbon atom(s), alkenyl with 2 to 6 carbon atoms, alkynyl with 2 to 6 carbon atoms, 3-to 8-membered cycloalkyl, 4- to 8-membered saturated heterocycle,
wherein each group may further be substituted by 1 or more substituent(s) independently selected from halogen, hydroxyl, alkyl with 1 to 6 carbon atom(s), alkoxy with 1 to 6 carbon atom(s), alkylsulfonyl with 1 to 6 carbon atom(s), amino optionally substituted by the same or different 1 or 2 alkyl(s) with 1 to 6 carbon atom(s), carbamoyl optionally substituted by the same or different 1 or 2 alkyl(s) with 1 to 6 carbon atom(s), and sulfamoyl optionally substituted by the same or different 1 or 2 alkyl(s) with 1 to 6 carbon atom(s);
(i) amino, carbamoyl or sulfamoyl which may be independently substituted by 1 or 2 substituent(s) described in the following (j) to (l):
(j) alkyl with 1 to 6 carbon atom(s), alkenyl with 2 to 6 carbon atoms, alkynyl with 2 to 6 carbon atoms, alkylcarbonyl with 2 to 6 carbon atoms, alkoxycarbonyl with 2 to 6 carbon atoms, alkylsulfonyl with 1 to 6 carbon atom(s), alkylsulfinyl with 1 to 6 carbon atom(s), alkenylcarbonyl with 3 to 6 carbon atoms, alkenyloxycarbonyl with 3 to 6 carbon atoms, alkenylsulfonyl with 2 to 6 carbon atoms, alkenylsulfinyl with 2 to 6 carbon atoms, alkynylcarbonyl with 2 to 6 carbon atoms, alkynyloxycarbonyl with 2 to 6 carbon atoms, alkynylsulfonyl with 2 to 6 carbon atoms, alkynylsulfinyl with 2 to 6 carbon atoms, 3- to 8-membered cycloalkyl, 3- to 8-membered cycloalkylcarbonyl, 3- to 8-membered cycloalkoxycarbonyl, 3- to 8-membered cycloalkylsulfonyl, 3- to 8-membered cycloalkylsulfinyl, 4- to 8-membered saturated heterocycle,
wherein each group may further be substituted by 1 or more substituent(s) independently selected from halogen, hydroxyl, alkoxy with 1 to 6 carbon atom(s), carboxy, alkoxycarbonyl with 2 to 6 carbon atoms, alkylsulfonyl with 1 to 6 carbon atom(s), amino optionally substituted by the same or different 1 or 2 alkyl(s) with 1 to 6 carbon atom(s), carbamoyl optionally substituted by the same or different 1 or 2 alkyl(s) with 1 to 6 carbon atom(s), and sulfamoyl with 1 to 6 carbon atom(s) optionally substituted by the same or different 1 or 2 alkyl(s);
(k) 6- to 10-membered aryl, 6- to 10-membered arylcarbonyl, 6- to 10-membered aryloxycarbonyl, 6- to 10-membered arylsulfonyl, 6- to 10-membered arylsulfinyl, 5- to 10-membered heteroaryl, 5- to 10-membered heteroarylcarbonyl, 5- to 10-membered heteroaryloxycarbonyl, 5- to 10-membered heteroarylsulfonyl, 5- to 10-membered heteroarylsulfinyl,
wherein each group may further be substituted by 1 or more substituent(s) independently selected from halogen, hydroxyl, alkyl with 1 to 6 carbon atom(s), alkoxy with 1 to 6 carbon atom(s), alkylsulfonyl with 1 to 6 carbon atom(s), amino optionally substituted by the same or different 1 or 2 alkyl(s) with 1 to 6 carbon atom(s), carbamoyl optionally substituted by the same or different 1 or 2 alkyl(s) with 1 to 6 carbon atom(s), and sulfamoyl optionally substituted by the same or different 1 or 2 alkyl(s) with 1 to 6 carbon atom(s);
(l) when 2 substituents of amino, carbamoyl and sulfamoyl are combined together with nitrogen atom to form 4- to 7-membered saturated nitrogen-containing heterocycle with 1 to 4 heteroatom(s) selected from 1 to 2 nitrogen(s), 0 to 1 oxygen and 0 to 1 sulfur,
wherein the saturated nitrogen-containing heterocycle may be substituted on any carbon atoms or nitrogen atoms by halogen, hydroxyl, alkyl with 1 to 6 carbon atom(s), alkoxy with 1 to 6 carbon atom(s), carboxyl, alkoxycarbonyl with 2 to 6 carbon atoms, alkylsulfonyl with 1 to 6 carbon atom(s), amino optionally substituted by the same or different 1 or 2 alkyl(s) with 1 to 6 carbon atom(s), carbamoyl optionally substituted by the same or different 1 or 2 alkyl(s) with 1 to 6 carbon atom(s), or sulfamoyl optionally substituted by the same or different 1 or 2 alkyl(s) with 1 to 6 carbon atom(s), where the substituent may be kept in chemically stable state; or a pharmaceutically acceptable salt thereof.

3. The adenine compound of either of claim 1 or 2, wherein R² is alkyl(s) with 1 to 4 carbon atom(s) in the formula (1), or a pharmaceutically acceptable salt thereof.

4. The adenine compound of claim 3, wherein R² is methyl, or a pharmaceutically acceptable salt thereof.

5. The adenine compound of claim 1, wherein R² is substituted alkyl with 2 to 6 carbon atoms in the formula (1), or a pharmaceutically acceptable salt thereof.

6. The adenine compound of claim 5, wherein R² is alkyl with 2 to 6 carbon atoms substituted by substituted or unsubstituted amino in the formula (1), or a pharmaceutically acceptable salt thereof.

7. The adenine compound of any one of claims 1 to 6, wherein R³ and R⁴ are independently hydrogen or alkyl with 1 to 3 carbon atom(s) in the formula (1), or a pharmaceutically acceptable salt thereof.

8. The adenine compound of any one of claims 1 to 7, wherein R⁵ is hydrogen or substituted or unsubstituted alkyl in the formula (1), or a pharmaceutically acceptable salt thereof.

9. The adenine compound of claim 8, wherein the substituent on the substituted alkyl in R⁵ is selected from alkoxycarbonyl with 2 to 5 carbon atoms, carboxy, hydroxyl, amino optionally substituted by the same or different 1 or 2 alkyl(s) with 1 to 6 carbon atom(s), carbamoyl and 6- to 10-membered aryl wherein the aryl may be substituted by 1 or more of the same or different substituent(s) independently selected from halogen; hydroxyl; alkyl with 1 to 6 carbon atom(s) or alkoxy with 1 to 6 carbon atom(s) each of which may be substituted by hydroxyl, alkoxy with 1 to 6 carbon atom(s) or amino optionally substituted by the same or different 1 or 2 alkyl(s) with 1 to 6 carbon atom(s); haloalkyl with 1 to 6 carbon atom(s); haloalkoxy with 1 to 6 carbon atom(s); and amino optionally substituted by the same or different 1 or 2 alkyl(s) with 1 to 6 carbon atom(s), or a pharmaceutically acceptable salt thereof.

10. The adenine compound of claim 1 selected from the following compounds:
methyl N-[2-(6-amino-2-butoxy-7,8-dihydro-8-oxo-9H-purin-9-yl)-ethyl]-glycine;
methyl (2S)-2-{[2-(6-amino-2-butoxy-8-oxo-7,8-dihydro-9H-purin-9-yl)-ethyl]-amino}-butanone;
methyl N-[2-(6-amino-2-butoxy-8-oxo-7,8-dihydro-9H-purin-9-yl)-ethyl]-L-alaninate;
methyl N-[2-(6-amino-2-butoxy-8-oxo-7,8-dihydro-9H-purin-9-yl)-ethyl]-2-methylalaninate;
methyl N-[2-(6-amino-2-butoxy-8-oxo-7,8-dihydro-9H-purin-9-yl)-ethyl]-D-valinate;
dimethyl N-[2-(6-amino-2-butoxy-8-oxo-7,8-dihydro-9H-purin-9-yl)-ethyl] -L-aspartate;
N²-[2-(6-amino-2-butoxy-8-oxo-7,8-dihydro-9H-purin-9-yl)-ethyl]-L-lysine methyl ester;
methyl (2S)-{[2-(6-amino-2-butoxy-8-oxo-7,8-dihydro-9H-purin-9-yl)-ethyl]-amino}(4-hydroxyphenyl)-acetate;
methyl N-[2-(6-amino-2-butoxy-8-oxo-7,8-dihydro-9H-purin-9-yl)-ethyl]-N-methyl glycinate;
methyl N-[3-(6-amino-2-butoxy-8-oxo-7,8-dihydro-9H-purin-9-yl)-propyl]-O-[3-(dimethylamino)propyl]-L-tyrosinate;
methyl N-[4-(6-amino-2-butoxy-8-oxo-7,8-dihydro-9H-purin-9-yl)butyl]-L-alaninate;
methyl N-[4-(6-amino-2-butoxy-8-oxo-7,8-dihydro-9H-purin-9-yl)butyl]-N-methyl glycinate;
N-[2-(6-amino-2-butoxy-8-oxo-7,8-dihydro-9H-purin-9-yl)-ethyl]-L-alanine;
N-[2-(6-amino-2-butoxy-8-oxo-7,8-dihydro-9H-purin-9-yl)-ethyl]-D-valine;
N-[2-(6-amino-2-butoxy-8-oxo-7,8-dihydro-9H-purin-9-yl)-ethyl]-glycine;
(2S)-2-{[2-(6-amino-2-butoxy-8-oxo-7,8-dihydro-9H-purin-9-yl)-ethyl]-amino}butane acid;
N-[2-(6-amino-2-butoxy-8-oxo-7,8-dihydro-9H-purin-9-yl)-ethyl]-2-methylalanine;
N-[2-(6-amino-2-butoxy-8-oxo-7,8-dihydro-9H-purin-9-yl)-ethyl]-N-methylglycine; or a pharmaceutically acceptable salt thereof.

11. A pharmaceutical composition comprising as an active ingredient the adenine compound of any one of claims 1 to 10, or a pharmaceutically acceptable salt thereof.

12. An agent for increasing TLR7 activity comprising as an active ingredient the adenine compound of any one of claims 1 to 10, or a pharmaceutically acceptable salt thereof.

13. An immune-regulating agent comprising as an active ingredient the adenine compound of any one of claims 1 to 10, or a pharmaceutically acceptable salt thereof.

14. A therapeutic or preventive agent for allergic disease, viral disease or cancer comprising as an active ingredient the adenine compound of any one of claims 1 to 10, or a pharmaceutically acceptable salt thereof.

15. A therapeutic or preventive agent for asthma, COPD, allergic rhinitis, allergic conjunctivitis, atopic dermatitis, cancer, hepatitis B, hepatitis C, HIV, HPV, bacterial infectious disease or dermatitis comprising as an active ingredient the adenine compound of any one of claims 1 to 10, or a pharmaceutically acceptable salt thereof.

16. A pharmaceutical composition for local administration comprising as an active ingredient the adenine compound of any one of claims 1 to 10, or a pharmaceutically acceptable salt thereof.
